# EUROPEAN PATENT SPECIFICATION

(11) **EP 2 331 106 B1**
(45) Date of publication and mention of the grant of the patent: **10.10.2012**
(21) Application number: 09785024.2
(22) Date of filing: 28.08.2009
(51) Int. Cl.: A61K 31/7072, C07H 19/073, C07H 19/067

(54) **NUCLEOSIDE ANALOGUES USEFUL AS POSITRON EMISSION TOMOGRAPHY (PET) IMAGING AGENTS**
NUKLEOSIDANALOGA ALS PET-BILDGEBUNGSMITTEL
ANALOGUES DE NUCLÉOSIDE UTILES EN TANT QU'AGENTS D'IMAGERIE DE TOMOGRAPHIE PAR ÉMISSION DE POSITRONS (PET)

(30) Priority: 01.09.2008 GB 0815831
(43) Date of publication of application: 15.06.2011
(73) Proprietor: Imperial Innovations Limited, London SW7 2AZ (GB)
(72) Inventor: ABOAGYE, Eric, Ofori, Du Cane Road London W12 0NN (GB); SMITH, Graham, Du Cane Road London W12 0NN (GB)
(74) Representative: Pilkington, Stephanie Joan
(86) International application number: PCT/GB2009/002096
(87) International publication number: WO 2010/023457

(56) References cited:
- EP-A- 1 270 017
- EP-A- 1 916 003
- WO-A-01/05439
- WO-A-2008/024826
- WO-A-2008/109080
- GHOSH PRADIP ET AL: "N-3-Substituted thymidine analogues III: radiosynthesis of N-3-[(4-[F-18]fluoromethyl-phenyl)butyl]th ymidine ([F-18]-FMPBT) and N-3-[(4-[F-18]fluoromethyl-phenyl)pentyl] thymidine ([F-18]-FMPPT) for PET" JOURNAL OF LABELLED COMPOUNDS AND RADIOPHARMACEUTICALS, JOHN WILEY, CHICHESTER, GB, vol. 50, no. 13-14, 1 November 2007 (2007-11-01), pages 1185-1191, XP002513298 ISSN: 0362-4803
- SIRION ET AL: "An efficient F-18 labeling method for PET study: Huisgen 1,3-dipolar cycloaddition of bioactive substances and F-18-labeled compounds" TETRAHEDRON LETTERS, ELSEVIER, AMSTERDAM, vol. 48, no. 23, 4 June 2007 (2007-06-04), pages 3953-3957, XP022069423 ISSN: 0040-4039

## Description

The present invention concerns compounds considered to be useful as positron emission tomography (PET) imaging agents that could be used to measure cell proliferation in *vivo.* The compounds are considered to be useful in prognosis and prediction of therapeutic response.

Nucleoside analogues have been used specifically for imaging proliferation due to their suitability as substrates for the cell cycle regulated protein TK1 (1), and in some cases their suitability to be incorporated into DNA (2). Such imaging agents are considered to be useful in measuring how rapidly cancer cells divide. Because this property is a hallmark of most cancers, such agents are considered potentially to have generic utility for diagnosis, prognosis and detection of treatment response. The current standard for imaging cell proliferation in mouse and human tumours is [¹⁸F]fluorothymidine (FLT; I) PET. Unlike the nature-identical nucleoside imaging agent, 2-[¹¹C]thymidine ([¹¹C]TdR (II), FLT is stable to metabolic degradation. Our group has shown that tumour FLT uptake in human breast cancer patients determined by PET strongly correlates with Ki-67 immunostaining (3). Studies in lung cancer patients by another group also showed high correlation of tumour FLT uptake with Ki-67 (4). Furthermore, we have shown that FLT-PET is able to image early response to both cytostatic and cytotoxic agents in mouse models of cancer and in patients (5-9). This makes FLT a promising agent for imaging cell proliferation and to image early response to treatment. The two major limitations of FLT-PET are low overall detection sensitivity, particularly for lowly proliferating tumours and poor radiosynthetic yield.

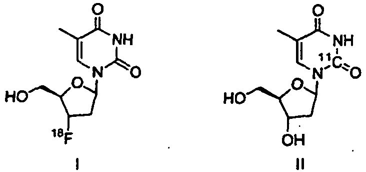

Our aim has been to develop a proliferation marker that is sensitive, specific and robust to metabolic degradation. We and others have shown that although more specific for imaging proliferation, the overall tumour FLT uptake is less sensitive compared to that of [¹⁸F]fluorodeoxyglucose (5, 10). This may be due to a number of factors including the relatively higher propensity of tumour cells for glycolysis compared to nucleoside synthesis, but also properties of FLT itself. FLT is taken up into cells by the nucleoside transporter system and phosphorylated by TK1; the substrate specificity of FLT for TK1 is much less than that of TdR and once phosphorylated, FLT-phosphate is not a good substrate for further phosphorylation and incorporation into DNA (5, 11). It is possible that in lowly proliferating cells nucleotidases can dephosphorylate FLT-phosphate leading to reduction of the PET signal. We consider that an analogue that is more irreversibly incorporated into DNA is desirable and might provide the high sensitivity required for detection of tumours with low proliferation index. Such an analogue would have to retain the robust metabolic stability of FLT which is due to substitution of a fluorine atom in the 3' position of the sugar ring. This substitution, however, is responsible for the reduction in affinity of FLT for TK1 (12). TK1 is known to tolerate minor modifications at the 5-position; recently it has been shown that the enzyme also tolerates bulky substitutions in the N-3 position (12).

EP 1916003 discloses labelled thymidine analogues which are particularly useful for position emission tomography (PET).

Our aim has also been to design probes that can be radiolabelled efficiently, preferably with fluorine-18 given its suitable properties for PET (t_{1/2} of 109.8 min, low positron energy and hence good resolution, and high photon flux); or other positron emitting or single photon emitting radioisotopes (for SPECT). The half-life of fluorine-18, for example, offers the ability to use the imaging agents at sites that lack an on-site cyclotron. Examples of suitable PET/SPECT isotopes include ¹¹C, ¹⁸F, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I, ²⁰¹TI.

There are several limitations of current methodologies for introducing fluorine-18 into biologically relevant molecules. Cu(I)-catalyzed 1,3-dipolar Huisgen cycloaddition of terminal alkynes to labelled azide derivatives, also known as the 'click reaction', to form [¹⁸F]-labelled triazoles is a flexible strategy that we consider offers the potential to overcome several of these limitations (e.g. functional group incompatibility) and often proceeds in very high radiochemical yield. Our work encompasses Huisgen cycloaddition using both azide and terminal alkyne functionalised prosthetic groups (Scheme 1).

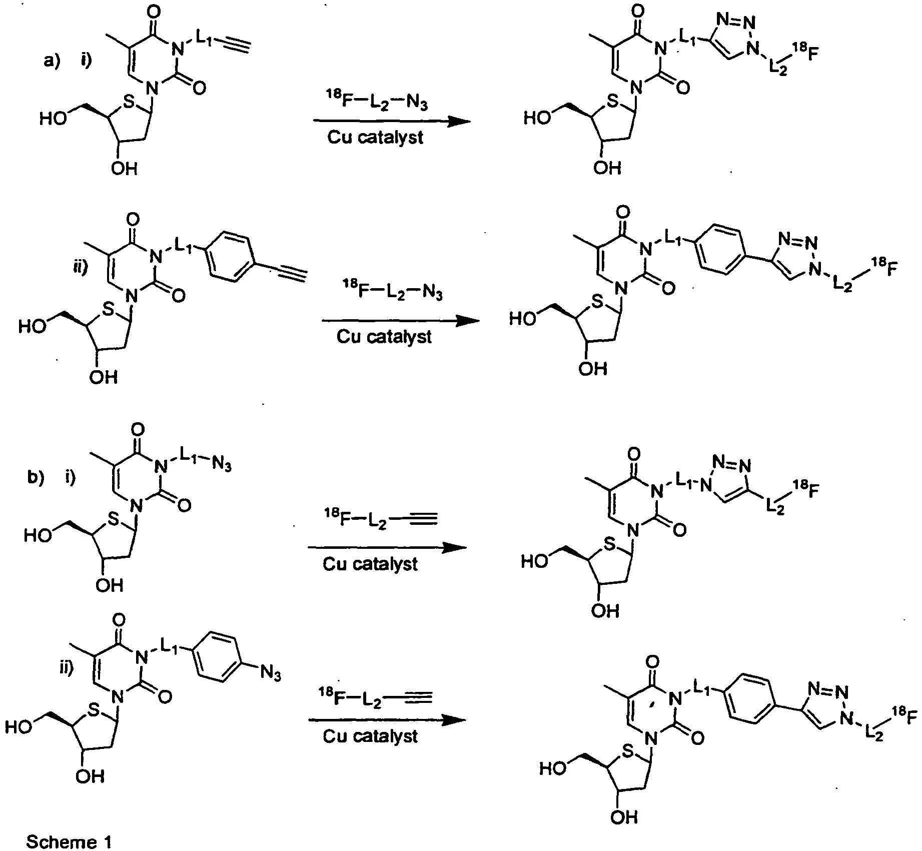

The linker groups L₁ and L₂ in Schemes 1, 2, 3 and 4 are each independently a C₁₋₃₀ hydrocarbon chain which may be branched or straight chain, although straight chain is typically preferred. The hydrocarbon chain may be optionally substituted with 1 to 15 heteroatoms such as oxygen, nitrogen or sulfur. The chain may also include alkenyl, alkynyl or cycloalkyl units. The cycloalkyl ring would consist of 3 to 12 carbon atoms, optionally substituted with 1 to 5 heteroatoms such as oxygen, nitrogen or sulfur. The linker unit may additionally comprise an aryl, polyaryl or heteroaryl unit. Aryl is defined as an aromatic ring of 5 or 6 core carbon atoms. Polyaryl refers to multiple aryl rings that are fused as in napthyl, or unfused, as in biphenyl. Heteroaryl refers to an aromatic ring of 5 or 6 carbon atoms in which one or more carbon atoms is substituted with a heteroatom, typically oxygen, nitrogen or sulfur. The heteroaryl unit can also be fused to another aryl or heteroaryl unit. The aryl, polyaryl or heteroaryl unit may be optionally substituted with one or more C₁₋₅ alkyl, C₃₋₆ alkenyl, C₃₋₆ alkynyl, halogen (fluorine, chlorine, bromine, iodine), -CF₃, nitro, amino, hydroxyl, aldehyde, COOC₁₋₅ alkyl, -OC₁₋₅ alkyl, CONHC₁₋₅ alkyl, -NHCOC₁₋₅ alkyl and -NHSO₂C₁₋₅ alkyl.

The reaction has recently been developed for peptide labelling (15). The reactions occur under mild conditions to produce stable products with size similar to an iodine atom and polarity similar to that of an amide. In addition to providing [¹⁸F]-labelled nucleosides, for example, click chemistry to provide ¹¹C-labelled triazoles has recently been developed. This can be used to provide [¹¹C]-labelled nucleosides as shown in Scheme 2.

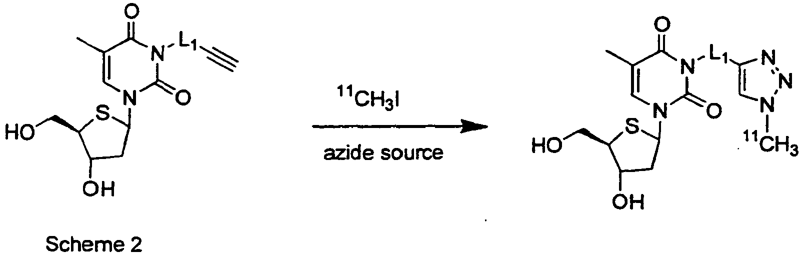

In a further alternative, a metal-chelating agent such as DOTA (1,4,7,10-tetraazacyclodecane-1,4,7,10-tetraacetic acid) or HYNIC (6-hydrazinopyridine-3-carboxylic acid) may be attached via a click chemistry cycloaddition as shown in Scheme 4 (for a DOTA appendage). Complexation for a DOTA appendage could then proceed as shown in Schemes 3 and 4. A HYNIC appendage may be used for, for example, technetium (^{94m}Tc, ^{99m}Tc) complexation.

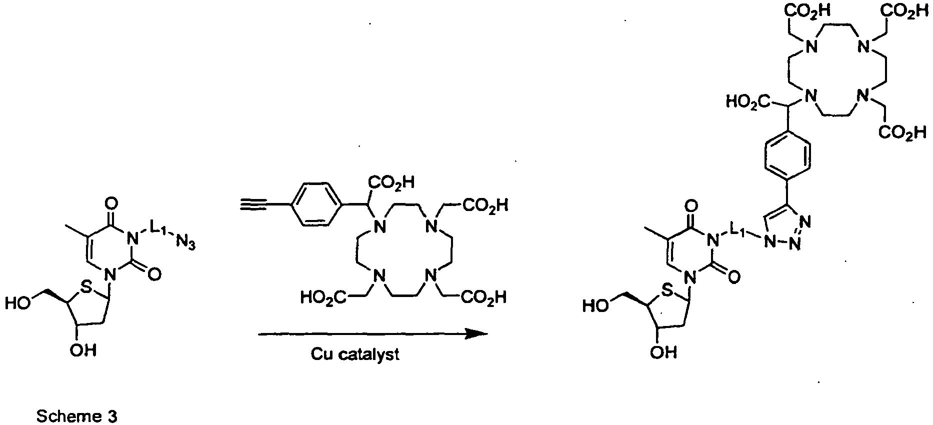

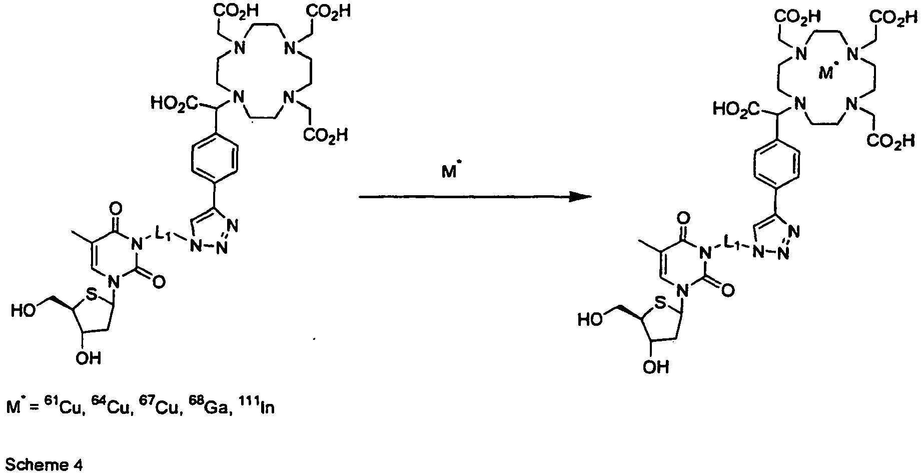

We have focused on developing "4'-thio-nucleosides" for example 4'-thio-thiothymidine or 4'-thio-2'-deoxyuridine analogues. It is now well recognised that 3' or 2' substitution of the sugar group with electronegative fluorine stabilizes the N-glycosidic bond to phosphorylases, 3' substitution in FLT and 2' substitution in [¹⁸F]fluoro(2'-deoxy-2'-β-arabinofuranosyl)thymidine (FMAU) being classical examples (2). The much lower low tolerance of TK1 for substitutions at the 2'-up position (12) is probably responsible for the poor cellular uptake of FMAU compared to FLT despite the former being more efficiently incorporated into DNA (11); both probes have much lower cellular uptake than radiolabelled TdR due to 3'/2' substitution (12). Furthermore, like the iodine analogue of FMAU, FIAU, the 2'-up substitution in FMAU is more efficiently phosphorylated by TK2 an enzyme that is not cell cycle regulated (16) and may account for the high uptake of FMAU in myocardium (17, 18). An alternative strategy to stabilize the N-glycosidic bond is through replacement of the furanose ring oxygen with sulphur, a method that has been used by Toyohara and co-workers to develop iodinated thiothymidine analogues (19-21). These thiothymidine analogues have similar conformation and *in vitro* phosphorylation and DNA incorporation kinetics as TdR. Whereas the original thiothymidine analogues of Toyohara were unsuitable for routine clinical PET, they demonstrated that with a suitable labelling approach, thiothymidine analogues would have an acceptable stability profile, as well as being incorporated into DNA hence increasing their tumour detection sensitivity. The current literature has structures that contain carbon-11 radiolabelled sulphur containing tracers (Toyohara J., Nucl. Med. Biol. 2008, 35, 67-74) or lodine-124/125 (Toyohara et al (2002) J Nucl Med 43(9), 1218-1226;Toyohara et al (2003) J Nucl Med 44, 1671-1676).

A first aspect of the invention provides a 4'-thio nucleoside that is a derivative of 4'-thiothymidine or 4'-thio-2'-deoxyuridine comprising a radioisotope, preferably a positron or single photon emitting radioisotope, or corresponding non-radioactive (stable) isotope attached via a triazole link to the N-3 position. Some examples are set out in Scheme 1 or Scheme 2 above. Alternatively the triazole link could be to a metal-chelating agent such as DOTA (and HYNIC), as set out in Schemes 3 and 4.

The term 4'-thio nucleoside will be well known to those skilled in the art. An exemplary structure is
1)

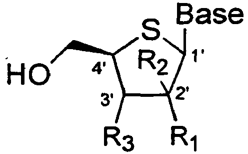

For the present invention R₁, R₂ and R₃ is H, OH or OCH₃.

An exemplary structure for the base is
2)

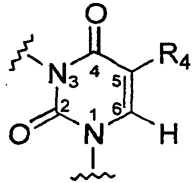

R₄ may be, for example, H or CH₃. R₄ may alternatively be a linear or branched alkyl group, for example a C2-C4 alkyl group, alkenyl, alkynyl, cyclic alkyl, for example C3-C7 cyclic alkyl group, or halogen. Alternatively R₄ may be a Sn- or Ge-functionalised solid support. Preferably R₄ is H or CH₃. The reaction schemes and molecules of the invention are illustrated herein by compounds in which R₄ is CH₃. It will, however, be appreciated by the reader that in these reaction schemes and molecules the CH₃ group could be replaced by another R₄ group as defined above, for example H.

The term triazole link will also be well known to those skilled in the art. It may be termed a 1,4 disubstituted 1,2,3-triazole. The structure may be represented as
3)

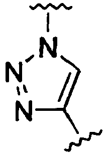

The 4'-thio nucleoside may be in the form of a salt. The salts which may be conveniently used for injection include physiologically acceptable base salts, for example, derived from an appropriate base, such as an alkali metal (eg sodium), alkaline earth metal (eg magnesium) salts, ammonium and NX₄⁺ (wherein X is C₁₋₄ alkyl) salts. Physiologically acceptable acid salts include hydrochloride, sulphate, mesylate, besylate, phosphate and glutamate.

Salts according to the invention may be prepared in conventional manner, for example by reaction of the parent compound with an appropriate base to form the corresponding base salt, or with an appropriate acid to form the corresponding acid salt.

The positron emitting radioisotope may be [¹⁸F] or its corresponding non-radioactive (stable) isotope, which is ¹⁹F.

Alternatively the positron or single photon emitting radioisotope may be ¹¹C, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I, or ²⁰¹TI. The radioisotope may alternatively be, for example, ³H, ¹⁴C, ³⁵S or ¹²⁵I, for example for laboratory investigations.

For ¹¹C there are two corresponding naturally occurring stable isotopes (¹²C and ¹³C). Heavier elements have multiple stable isotopes, as will be known to those skilled in the art.

In embodiments, the 4'-thio nucleoside can have the structure shown below where the linker groups L₁ and L₂ are as previously defined above. R is a reporter moiety which comprises a PET or SPECT radionuclide selected from the list above. Metallic radionuclides can be incorporated by use of a metal-chelating agent such as DOTA (or similar bifunctional aminocarboxylate chelator) or HYNIC.

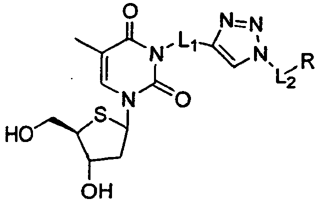

In embodiments, the 4'-thio nucleoside can have the structure shown below where the linker groups L₁ and L₂ are as previously defined above. R is a reporter moiety which comprises a PET or SPECT radionuclide selected from the list above. Metallic radionuclides can be incorporated by use of a metal-chelating agent such as DOTA (or similar bifunctional aminocarboxylate chelator) or HYNIC.

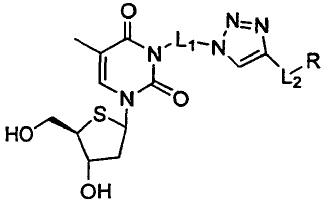

The following structures represent particularly preferred embodiments of the 4'-thio nucleoside of the invention:

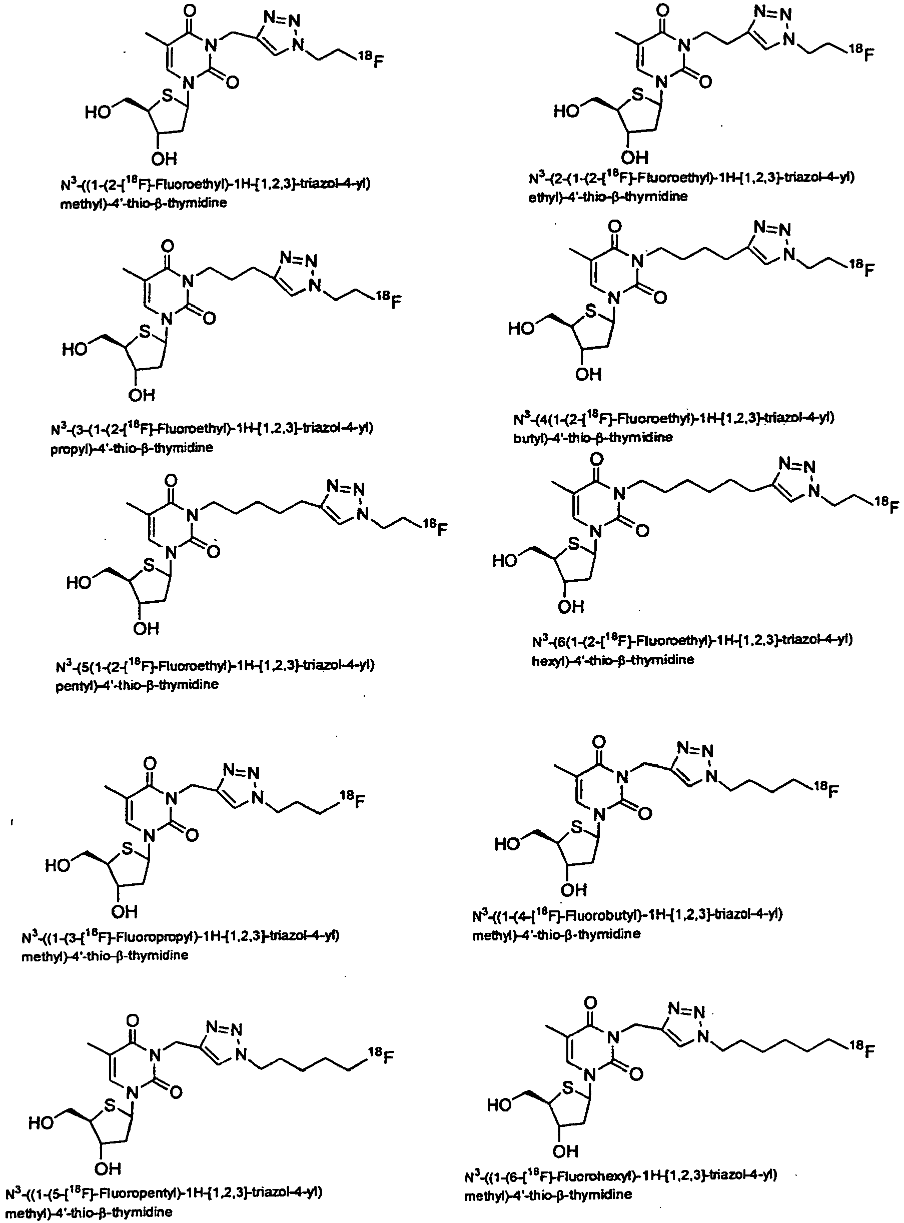

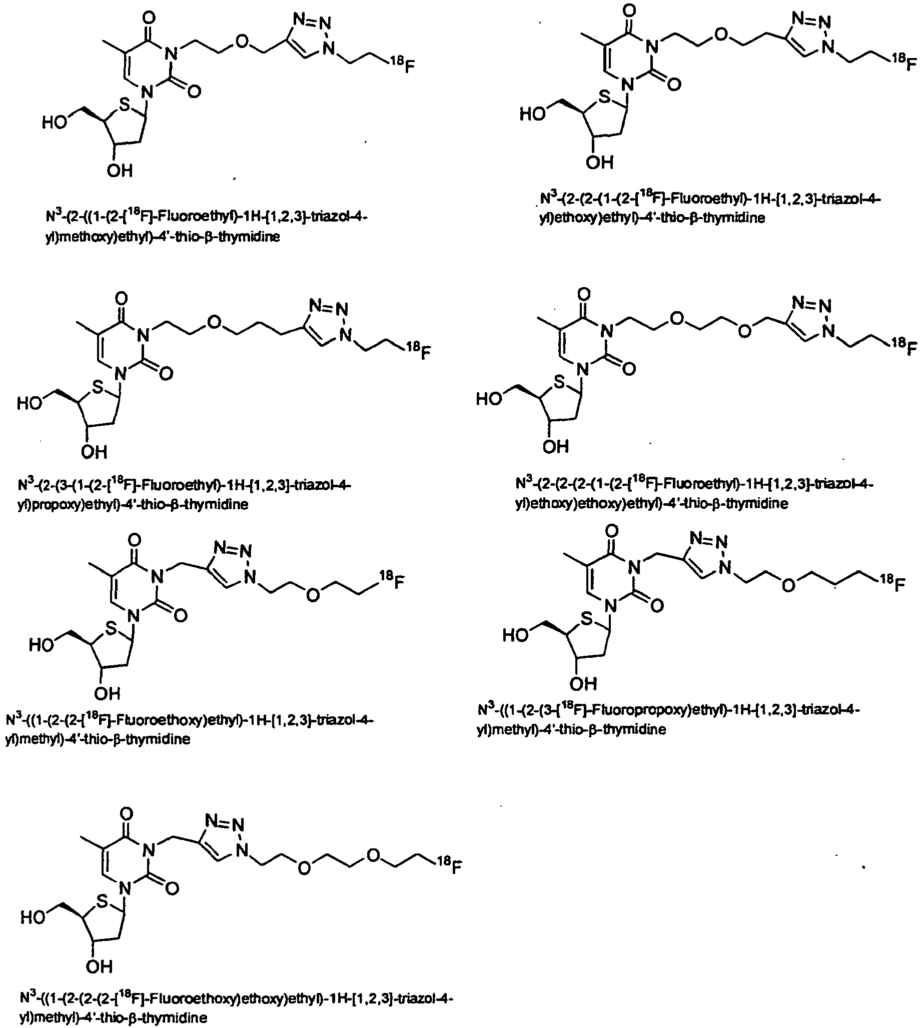

In further embodiments, the 4'-thio nucleoside can have the structure set out below where the PET/SPECT radioisotope (M*) e.g. ⁶¹Cu ⁶⁴Cu, ⁶⁷Cu, ⁶⁷Ga, ⁶⁸Ga, ¹¹¹In, ²⁰¹TI, ^{94m}Tc, ^{99m}Tc (or other radioisotope mentioned above, for example ³H, ¹⁴C or ¹²⁵I) is complexed by a suitable chelating agent and the chelating agent is attached to the thionucleoside via a triazole linkage. Suitable chelating agents include DOTA (or similar bifunctional aminocarboxylate chelator) and HYNIC. L₁ is as described above.

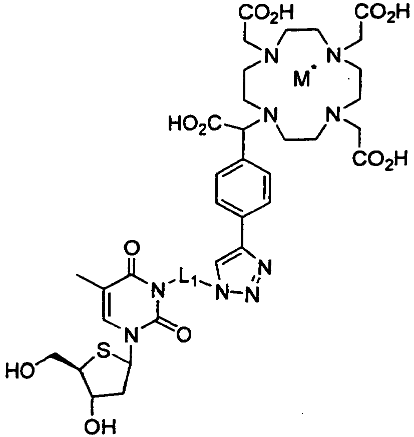

The following structures represent further particularly preferred embodiments of the 4'-thio nucleoside of the invention:-

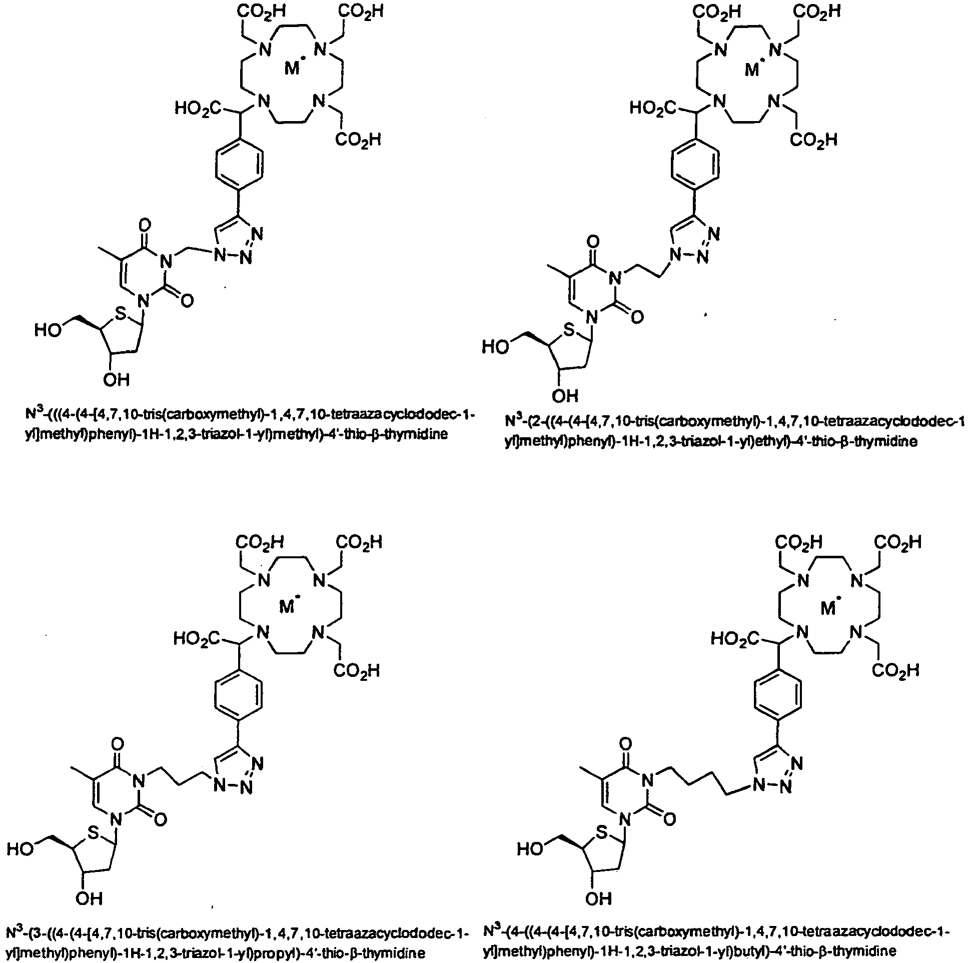

M* is a radiometallic reporter, which may be selected from following examples:- ⁶¹Cu, ⁶⁴Cu, ⁶⁷Cu, ⁶⁷Ga, ⁶⁸Ga, ^{94m}Tc, ^{99m}Tc, ¹¹¹In, ²⁰¹TI, ³H, ¹⁴C or ¹²⁵I.

A further aspect of the invention provides a method for preparing a 4'-thio nucleoside of the invention comprising the step of exposing a derivative of 4'-thiothymidine or 4'-thio-2'-deoxyuridine comprising a terminal alkyne attached via the N-3 position to a radiolabelled (or corresponding non-radiolabelled) compound comprising an azide group; or the step of exposing a derivative of 4'-thiothymidine or 4'-thio-2'-deoxyuridine comprising an azide group attached via the N-3 position to a radiolabelled (or corresponding non-radiolabelled) compound comprising a terminal alkyne group. The radiolabel is as discussed above in relation to preceding aspects of the invention, for example a positron or single photon emitting radioisotope.

In an embodiment of the method of the preceding aspect, the exposing step is carried out in the presence of a metal catalyst. The metal catalyst may be any suitable catalyst as would be appreciated by the skilled person. In a preferred embodiment the catalyst is a copper-containing catalyst. The catalyst may be copper powder. It is particularly preferred that the catalyst is copper sulfate/sodium ascorbate. It is envisaged that a Monodentate phosphoramidite ligand (see Campbell-Verduyn et al, (2009) Chem Commun (16):2139-41) or bathophenanthroline may be used as additives to the copper sulphate/sodium acsorbate catalyst system.

In a further embodiment of the method of the preceding aspect, the exposing step is carried out at a temperature of between 0 and 150 °C. It is preferred that the exposing step is carried out at or above ambient temperature. Thus, the exposing step may be a carried out at between 10 and 100 °C, for example, between 15 and 95 °C, between 20 and 90 °C or between 25 and 85 °C. In a particularly preferred embodiment, the exposing step is carried out at 85 °C.

In an embodiment the nucleoside is attached to a solid support through one or both of the hydroxyl groups on the sugar ring, as shown below. In this case R is a precursor group comprising the azide group or terminal alkyne for reaction to give the final radiolabelled compound, which could then be cleaved from the solid support.

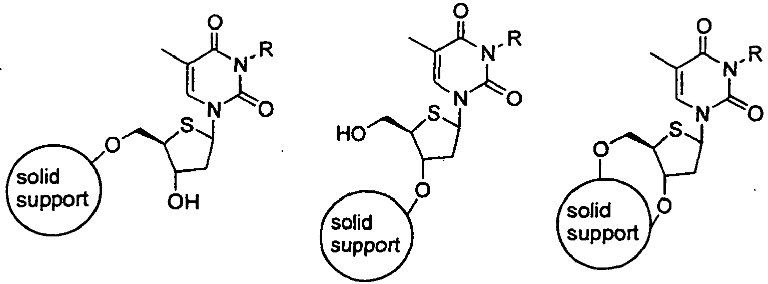

The solid support may be any suitable solid phase resin which is functionalised with an alkyl, trityl or acyl group. Ideally the resin should experience swelling in the solvent of choice. Examples of suitable resins include polymers such as polystyrene, polyamide, polyacrylamide or glass or silicon coated with a polymer. The solid phase resin may be in the form of small discrete particles such as beads or coated to the inner surface of a cartridge or on the lining of a reaction vessel.

A further aspect of the invention provides a pharmaceutical composition comprising a 4'-thio nucleoside of the invention and a pharmaceutically acceptable carrier. The carrier may be, for example, 5% ethanol in saline, but other carriers may also be used.

The compounds of the invention may normally be administered orally or by any parenteral route, in the form of a pharmaceutical formulation comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

In human therapy, the compounds of the invention can be administered alone but will generally be administered in admixture with a suitable pharmaceutical excipient diluent or carrier selected with regard to the intended route of administration and standard pharmaceutical practice.

For example, the compounds of the invention can be administered orally, buccally or sublingually in the form of tablets, capsules, ovules, elixirs, solutions or suspensions, which may contain flavouring or colouring agents, for immediate-, delayed- or controlled-release applications. The compounds of invention may also be administered via intracavernosal injection.

The compounds of the invention can also be administered parenterally, for example, intravenously, intra-arterially, intraperitoneally, intrathecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

For veterinary use, a compound of the invention is administered as a suitably acceptable formulation in accordance with normal veterinary practice and the veterinary surgeon will determine the dosing regimen and route of administration which will be most appropriate for a particular animal.

A further aspect of the invention provides a 4'-thio nucleoside of the invention for use in medicine.

A further aspect of the invention provides a 4'-thio nucleoside of the invention for use in aiding imaging, prognosis, diagnosis or analysis of response to treatment of a proliferative disease. A still further aspect of the invention provides the use of a 4'-thio nucleoside of the invention in the manufacture of a medicament for use in imaging, prognosis, diagnosis, selection for treatment or analysis of response to treatment of a proliferative disease.

The proliferative disease is typically cancer including all solid tumours, lymphomas and leukaemia. Other proliferative diseases include but are not limited to rheumatoid arthritis, endometrial hyperplasia, vascular restinosis, and sclerosis. Situations in which the derivatives of the present invention will be useful will be apparent to those skilled in the art by analogy with other positron imaging agents or experimental imaging agents.

A further aspect of the invention provides a kit of parts comprising a derivative of 4'-thiothymidine or 4'-thio-2'-deoxyuridine comprising a terminal alkyne or azide attached via the N-3 position and a radiolabelled (or corresponding non-radiolabelled) compound comprising an azide group or terminal alkyne group.

In a yet further aspect the invention provides for the use of a 4'-thio nucleoside of the invention for *in vitro* analysis of proliferation and anti-proliferative activity.

The invention is now described in more detail by reference to the following, non-limiting, Examples.

### References

1. Barthel H, Perumal M, Latigo J, He Q, Brady F, Luthra SK, et al. The uptake of 3'deoxy-3'-[18F]Fluorothymidine into L5178Y tumors in vivo is dependent on thymidine kinase 1 protein and ATP levels. Eur. J. Nucl. Med. Mol. Imaging 2005;32:257-263.
2. Kenny LM, Aboagye EO, Price PM. Positron emission tomography imaging of cell proliferation in oncology. Clin. Oncol. 2004;16:176-185.
3. Kenny LM, Vigushin DM, Al-Nahhas A, Osman S, Luthra SK, Shousha S, et al. Quantification of cellular proliferation in tumor and normal tissues of patients with breast cancer by [18F]fluorothymidine-positron emission tomography imaging: evaluation of analytical methods. Caner Res 2005;65:10104-10112.
4. Buck AK, Halter G, Schirrmeister H, Kotzerke J, Wurzinger I, Glatting G, et al. Imaging proliferation in lung tumors with PET: 18F-FLT versus 18F-FDG. J. Nucl. Med. 2003;44:1426-1431.
5. Barthel H, Cleij MC, Collingridge DR, Hutchinson OC, Osman S, He Q, et al. 3'-deoxy-3'-[18F]Fluorothymidine as a new marker for monitoring tumor response to anti-proliferative therapy in vivo with positron emission tomography. Cancer Res 2003;3:3791-3798.
6. Kenny L, Coombes RC, Vigushin DM, Al-Nahhas A, Shousha S, Aboagye EO. Imaging early changes in proliferation at 1 week post chemotherapy: a pilot study in breast cancer patients with 3'-deoxy-3'-[(18)F]fluorothymidine positron emission tomography. Eur. J. Nucl. Med. Mol. Imaging 2007;Epub [Ahead of print].
7. Leyton J, Alao JP, Da Costa M, Stavropoulou AV, Latigo JR, Perumal M, et al. In vivo biological activity of the histone deacetylase inhibitor LAQ824 is detectable with 3'-deoxy-3'-[18F]fluorothymidine positron emission tomography. Cancer Res. 2006;66:7621-7629.
8. Leyton J, Latigo JR, Perumal M, Dhaliwal H, He Q, Aboagye E. Early detection of tumour response to chemotherapy by 3'deoxy-3'-[18F]Fluorothymidine positron emission tomography: the effect of cisplatin on a fibrosarcoma tumour model in vivo. Cancer Res. 2005;65:4202-4210.
9. Leyton J, Lockley M, Aerts JL, Baird SK, Aboagye EO, Lemoine NR, et al. Quantifying the activity of adenoviral E1A CR2 deletion mutants using renilla luciferase bioluminescence and 3'-deoxy-3'-[18F]fluorothymidine positron emission tomography imaging. Cancer Res. 2006;66:9178-9185.
10. Buck AK, Schirrmeister H, Hetzel M, von Der Heide M, Halter G, Glatting G, et al. 3-deoxy-3-[(18)F]fluorothymidine-positron emission tomography for non-invasive assessment of proliferation in pulmonary nodules. Cancer Res. 2002;62:3331-3334.
11. Grierson JR, Schwartz JL, Muzi M, Jordan R, Krohn KA. Metabolism of 3'-deoxy-3'[F-18]fluorothymidine in proliferating A549 cells: validation for positron emission tomography. Nucl. Med. Biol. 2004;31:829-837.
12. Bandyopadhyaya AK, Johnsamuel J, Al-Madhoun AS, Eriksson S, Tjarks W. Comparative molecular field analysis and comparative molecular similarity indices analysis of human thymidine kinase 1 substrates. Biorg. Med. Chem. 2005;13:1681-1689.
13. Nicolas F, De Sousa G, Thomas P, Placidi M, Lorenzon G, Rahmani R. Comparative metabolism of 3'-azido-3'-deoxythymidine in cultured hepatocytes from rats, dogs and humans. Drug Metab. Disp. 1995;23:308-313.
14. Rajaonarison JF, Lacarelle B, De Sousa G, Catalin J, Rahmani R. In vitro glucuronidation of 3'-azido-3'-deoxythymidine by human liver. Role of UDP-glucuronosyltransferase 2 form. Drug Metab. Disp. 1991;19:809-815.
15. Glaser M, Arstad E. "Click Labeling" with 2-[(18)F]Fluoroethylazide for Positron Emission Tomography. Bioconjugate Chem. 2007;18:989-993.
16. Wang J, Eriksson S. Phosphorylation of the anti-hepatitis B nucleoside analog 1-(2'-deoxy-2'fluoro-1-b-D-ribofuranosyl)-5-iodouracil (FIAU) by human cytosolic and mitochondrial thymidine and implications for cytotoxicity Antimicrob. Agents Chemother 1996;40:1555-1557.
17. Sun H, Mangner TJ, Collins JM, Muzik O, Douglas K, Shields AF. Imaging DNA synthesis in vivo with 18F-FMAU and PET. J. Nucl. Med. 2005;46:292-296.
18. Sun H, Sloan A, Mangner TJ, Vaishampayan U, Muzik O, Collins JM, et al. Imaging DNA synthesis with [18F]FMAU and positron emission tomography in patients with cancer. Eur. J. Nucl. Med. Mol. Imaging 2005;32:15-22.
19. Toyohara J, Gogami A, Hayashi A, Yonekura Y, Fujibayashi Y. Pharmacokinetics and metabolism of 5-125I-iodo-4'-thio-2'-deoxyuridine in rodents. J. Nucl. Med. 2003;44:1671-1676.
20. Toyohara J, Hayashi A, Sato M, Tanaka H, Haraguchi K, Yoshimura Y, et al. Rationale of 5-(125)I-iodo-4'-thio-2'-deoxyuridine as a potential iodinated proliferation marker. J. Nucl. Med. 2002;43:1218-1226.
21. Toyohara J, Kumata K, Fukushi K, Irie T, Suzuki K. Evaluation of 4'-[methyl-14C]thiothymidine for in vivo DNA synthesis imaging. J. Nucl. Med. 2006;47:1717-1722.

**Figure 1****. HPLC profiles for copper sulfate and copper powder catalysed click radiochemistry reactions.** A: cold standard (**5b**), B and C: copper sulfate catalysed reaction, D and E: analysis of purified product (eluted from C18 cartridge with ethanol, no PBS added) from copper sulfate reaction. HPLC conditions:- ACE 10 x 100 mm column, 5-70 % methanol in 15 min, 3 ml/min, UV 254 nm.
**Figure 2****. Proportion of radiotracer incorporated into the acid insoluble fraction (mainly DNA) of HCT116 cells.**
**Figure 3****. PET images of [18F]FTT in HCT116 tumour (T, arrowed) bearing mice.**
**Figure 4****. Time versus radioactivity curves obtained from region of interest analysis of the PET data from HCT116 tumour bearing mice.**
**Figure 5****. Selected radio-HPLC chromatograms from the metabolite study described in Example 3.** (A) Control [¹⁸F]FTT injectate; (B) Plasma 30 mins; (C) Tumour 30 mins; (D) Liver 30 mins; (E) Kidney 30 mins; (F) Urine 30 mins.

### Example 1

Compound 2b in Scheme 5 (references 1, 2 below) was synthesized according to a modification of the method by Inoue and Naka (References 1 & 2 below). Compounds **2a/2b** were then derivatized to make them amenable for PET radiolabeling. One example of this was to focus on the 3+2 Huisgen cycloaddition or "click chemistry" reaction to give **5a/5b,** the pathway to which is shown in Scheme 5 below. This approach is attractive because of the excellent functional group tolerance exhibited by the reaction, removing the need for a protecting group strategy and thus improving radiosynthesis time.

### References

1. Inoue, N., Kaga, D., Minakawa, N., and Matsuda, A. Practical Synthesis of 2'-Deoxy-4'-thioribonucleosides: Substrates for the Synthesis of 4'-ThioDNA. Journal of Organic Chemistry, 70: 8597-8600, 2005.
2. Naka, T., Minakawa, N., Abe, H., Kaga, D., and Matsuda, A. The Stereoselective Synthesis of 4'-ß-Thioribonucleosides via the Pummerer Reaction. Journal of the American Chemical Society, 122: 7233-7243, 2000.

### Synthesis

Our synthetic methodology is summarised in Scheme 5 below. Alkylation of siloxane protected nucleosides **2a/2b** with propargyl bromide gave the *N*-alkylated nucleosides **3** 94 and 89% yield respectively. Cycloaddition with 2-fluoroethyl azide ('click' chemistry) gave the triazoles **4** before silyl deprotection using tetrabutylammonium fluoride (TBAF) to give the final 'cold' analogues **5**. The lower yield for the deprotection step (~50%) is likely simply the result of some material being leftover in the mother liquor from the recrystallisation.

The reaction sequence of cycloaddition followed by deprotection was found to be preferable to the alternative deprotection/cycloaddition sequence owing to difficulties encountered in isolating nucleoside **5a** from the cycloaddition reaction mixture when the click chemistry reaction was attempted using 6a.

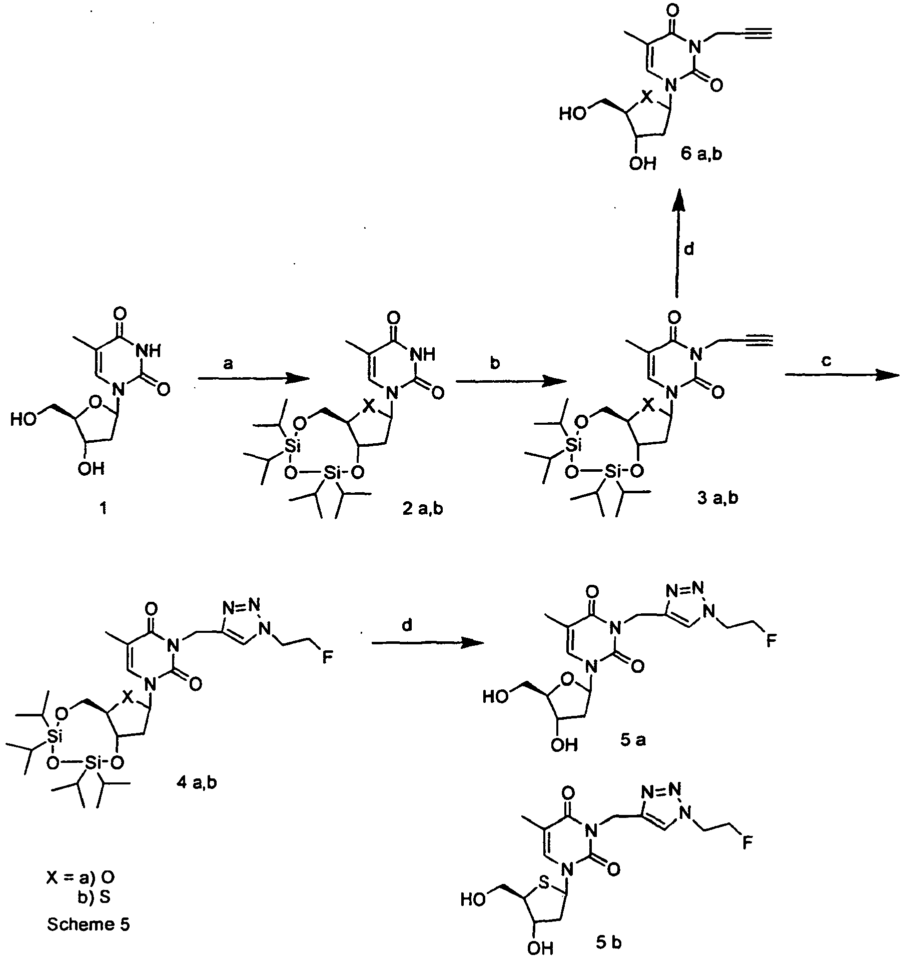

*Reagents and Conditions:* a) 1,3-dichloro-1,1,3,3-tetraisopropyldisiloxane, pyridine, rt, 40 h; b) propargyl bromide, K₂CO₃, DMF, rt, 48 h; c) 2-fluoroethylazide, CuSO₄, ascorbic acid, DMF, rt, 3 h; d) TBAF, THF, acetic acid, 0°C, 1.5 h.

### Experimental

Reagents and solvents were purchased from Sigma-Aldrich (Gillingham, United Kingdom) and used without further purification. Potassium carbonate was stored in a vacuum dessicator over phosphorus pentoxide. All reactions were carried out under argon unless otherwise stated. Flash chromatography was carried out using Davisil neutral silica (60 A, 60-200 micron, Fisher Scientific, Loughborough, UK), solvent mixtures are quoted as volume/volume. ¹H NMR Spectra were obtained on a Bruker Avance 600 MHz NMR machine and spectra are referenced to residual solvent. Coupling constants (*J*) are given in Hertz (Hz). Mass spectra were obtained in positive electrospray ionisation mode on a Waters Micromass LCT Premier. Melting points were determined in capillary tubes on a Stuart Scientific SMP1 melting point apparatus and are uncorrected. Solvent mixtures for thin layer chromatography (TLC) are quoted as volume/volume and samples were developed on aluminium backed neutral silica plates (0.2 mm thickness) (Fluka, Seelze, Germany).

4'-Thiothymidine 2b was synthesised according to the method of Inoue⁵ by Creative Chemistry (Uxbridge, Middlesex, UK) and used as supplied. Analysis by NMR indicated consistency with the previously reported structure.

### 3',5'-O-(1,1,3,3-Tetraisopropyldisiloxane-1,3-diyl)thymidine (2a)

To a solution of thymidine (1.21 g, 5 mmol) in dry pyridine (12 mL) was added 1,3-dichloro-1,1,3,3-tetraisopropyldisiloxane (1.74 g, 5.5 mmol) and the resulting solution stirred under argon at ambient temperature. After 40 h bulk solvent was removed in vacuo to give a colourless residue. Trituration with hexane gave a white powder that was used without further purification.

### 3',5'-O-(1,1,3,3-Tetraisopropyldisiloxane-1,3-diyl)-N³-(2-propynyl)thymidine (3a).

To a solution of 2a (0.73 g, 1.5 mmol) in dry DMF (10 mL) was added K₂CO₃ (0.41 g, 3 mmol) followed by propargyl bromide (80 wt. % in toluene) (0.71 g, 6 mmol) and the resulting mixture stirred under argon at ambient temperature. After 48 h TLC indicated complete conversion of **2a** and the mixture was poured onto 10% aq. citric acid (20 mL) and extracted with EtOAc (3 × 15 mL) and dried over Na₂SO₄. Column chromatography (3:1 hexanes/ethyl acetate) afforded the title compound as a colourless oil (0.74 g, 94 %). ¹H NMR (600 MHz, CDCl₃) δ 7.44 (d, *J* = 1.2 Hz, 1 H), 6.12 (d, *J* = 7.2 Hz, 1 H), 4.71 (m, 2 H), 4.50-4.46 (m, 1 H), 4.12 (dd, *J* = 2.4 Hz, *J* = 7.2 Hz, 1 H), 4.02 (dd, *J* = 3 Hz, *J* = 7.2 Hz, 1 H), 3.75 (dt, *J* = 8.4 Hz, *J* = 2.4 Hz, 1 H), 2.53-2.48 (m, 1 H), 2.27-2.23 (m, 1 H), 2.17 (t, *J* = 2.4 Hz, 1 H), 1.95 (d, *J* = 1.2 Hz, 3 H), 1.10-0.92 (m, 28 H).

### 3',5'-O-(1,1,3,3-Tetraisopropyldisiloxane-1,3-diyl)-N³-(2-propynyl)-4'-thio-β-thymidine (3b).

To a solution of **2b** (1.00 g, 2 mmol) in dry DMF (12 mL) was added K₂CO₃ (0.55 g, 4 mmol) followed by propargyl bromide (80 wt. % in toluene) (0.95 g, 8 mmol) and the resulting mixture stirred under argon at ambient temperature. After 48 h TLC indicated complete conversion of **2b** and the mixture was poured onto 10% aq. citric acid (30 mL) and extracted with EtOAc (3 × 15 mL) and dried over Na₂SO₄. Column chromatography (3:1 hexanes/ethyl acetate) afforded the title compound as a colourless oil (0.96 g, 89 %). ¹H NMR (600 MHz, CDCl₃) δ 7.89 (d, *J* = 1.8 Hz, 1 H), 6.11 (d, *J* = 7.2 Hz, 1 H), 4.72 (d, *J* = 2.4 Hz, 2 H), 4.48-4.44 (m, 1 H), 4.13 (dd, *J* = 3 Hz, *J* = 6.6 Hz, 1 H), 3.95 (dd, *J* = 1.8 Hz, *J* = 6.6 Hz, 1 H), 3.33 (dt, J = 9 Hz, *J* = 1.8 Hz, 1 H), 2.51-2.46 (m, 1 H), 2.28-2.24 (m, 1 H), 2.17 (t, *J* = 2.4 Hz, 1 H), 1.97 (d, *J* = 1.8 Hz, 3 H), 1.14-0.91 (m, 28 H).

### 3',5'-O-1,1,3,3-Tetraisopropyldisiloxane-1,3-diyl)-N³-((1-(2-fluoroethyl)-1H-[1,2,3]-triazol-4-yl)methyl)thymidine (4a).

To a solution of **3a** (104 mg, 0.2 mmol) in dry DMF (1 mL) was added ascorbic acid (36 mg, 0.2 mmol) in water (0.3 mL) followed by copper sulfate (25 mg, 0.1 mmol) in water (0.3 mL) followed by 2-fluoroethylazide (22 mg, 0.24 mmol) in dry DMF (1 mL) and the resulting mixture stirred under argon. After 3 h TLC indicated complete conversion of **3a** and the mixture was poured onto 10% aq. citric acid (10 mL) and extracted with EtOAc (3 × 10 mL) and dried over Na₂SO₄. Column chromatography (95:5 DCM:MeOH) gave the desired product as a colourless oil (93 mg, 76 %). ¹H NMR (600 MHz, CDCl₃) δ 7.71 (s, 1 H), 7.41 (s, 1 H), 6.12 (d, *J* = 7.2 Hz, 1 H), 5.27 (d, *J* = 19.2 Hz, 1 H), 5.25 (d, *J* = 19.2 Hz, 1 H), 4.77 (dt, *J* = 46.8 Hz, *J* = 4.8 Hz, 2 H), 4.62 (dt, *J* = 27 Hz, *J* = 4.8 Hz, 2 H), 4.49-4.45 (m, 1 H), 4.11 (dd, *J* = 2.4 Hz, *J* = 7.2 Hz, 1 H), 4.01 (dd, *J* = 3 Hz, *J* = 7.2 Hz, 1 H), 3.74 (dt, *J* = 7.8 Hz, J = 2.4 Hz, 1 H), 2.51-2.46 (m, 1 H), 2.25-2.21 (m, 1 H), 1.94 (s, 3 H), 1.10-0.92 (m, 28 H).

### 3',5'-O-(1,1,3,3-Tetraisopropyldisiloxane-1,3-diyl)-N³-((1-(2-fluoroethyl)-1H-[1,2,3]-triazol-4-yl)methyl)-4'-thio-β-thymidine (4b).

The thionucleoside **4b** was prepared according to the procedure for **4a** except using **3b** to give a colourless oil (101 mg, 80 %). ¹H NMR (600 MHz, CDCl₃) δ 7.85 (s, 1 H), 7.71 (s, 1 H), 6.12 (d, *J* = 7.8 Hz, 1 H), 5.26 (s, 2 H), 4.76 (dt, *J* = 46.8 Hz, *J* = 4.8 Hz, 2 H), 4.62 (dt, *J* = 26.4 Hz, *J* = 4.8 Hz, 2 H), 4.48-4.44 (m, 1 H), 4.12 (dd, *J* = 3 Hz, *J* = 6.6 Hz, 1 H), 3.96-3.94 (m, 1 H), 3.33-3.31 (m, 1 H), 2.50-2.42 (m, 1 H), 2.26-2.23 (m, 1 H), 1.94 (s, 3 H), 1.14-0.91 (m, 28 H).

### N³-((1-(2-Fluoroethyl)-1H-[1,2,3]-triazol-4-yl)methyl)thymidine (5a).

To a mixture of **4a** (92 mg, 0.15 mmol) and acetic acid (5 µL) cooled in an ice bath was added tetrabutylammonium fluoride (1.0 M in THF) (0.3 mL, 0.3 mmol). After 1.5 h TLC indicated complete conversion of **4a** and bulk solvent was removed in vacuo. Column chromatography (95:5 DCM:MeOH) yielded a colourless oil. Recrystallisation from EtOH gave the title compound as a white solid (21 mg, 35 %). HRMS (ESI) = 370.1521 (M + H)⁺. Calcd. for C₁₅H₂₁N₅O₅F 370.1527. ¹H NMR (600 MHz, CD₃CN) δ 7.73 (s, 1 H), 7.63 (d, *J* = 1.2 Hz, 1 H), 6.23 (t, *J* = 7.2 Hz, 1 H), 5.13 (d, *J* = 15 Hz, 1 H), 5.12 (d, *J* = 15 Hz, 1 H), 4.77 (dt, *J* = 46.8 Hz, *J* = 4.8 Hz, 2 H), 4.60 (dt, *J* = 27.6 Hz, *J* = 4.8 Hz, 2 H), 4.36-4.33 (m, 1 H), 3.83 (q, *J* = 3.6 Hz, 1 H), 3.73-3.65 (m, 2 H), 3.32 (d, *J* = 4.2 Hz, 1 H), 3.13 (t, *J*= 5.4 Hz, 1 H), 2.21-2.16 (m, 2 H), 1.87 (d, *J*= 1.2 Hz, 3 H).

### N³-((1-(2-Fluoroethyl)-1H-[1,2,3]-triazol-4-yl)methyl)-4'-thio-β-thymidine (5b)

The thionucleoside **5b** was prepared according to the procedure for **5a** except using **4b** to give a colourless oil. Recrystallisation from EtOH afforded a white solid (32 mg, 55 %). HRMS (ESI) = 386.1307 (M + H)⁺. Calcd. for C₁₅H₂₁N₅O₄FS 386.1298. ¹H NMR (600 MHz, CD₃CN) δ 7.77 (d, *J* = 1.2 Hz, 1 H), 7.73 (s, 1 H), 6.38 (t, *J* = 6.6 Hz, 1 H), 5.12 (s, 2 H), 4.77 (dt, *J* = 46.8 Hz, *J* = 4.8 Hz, 2 H), 4.60 (dt, *J* = 27.6 Hz, *J* = 4.8 Hz, 2 H), 4.44-4.42 (m, 1 H), 3.72-3.69 (m, 2 H), 3.37-3.34 (m, 2 H), 3.25 (t, *J* = 5.4 Hz, 1 H), 2.28-2.26 (m, 1 H), 2.21-2.16 (m, 1 H), 1.90 (d, *J* = 1.2 Hz, 3 H).

### N³-(2-Propynyl)thymidine (6a).

To a mixture **3a** (0.73 g, 1.4 mmol) and acetic acid (0.17 g, 2.8 mmol) cooled in an ice bath was added tetrabutylammonium fluoride (1.0 M in THF) (2.8 mL, 2.8 mmol). After 1.5 h TLC indicated complete conversion of **3a** and bulk solvent was removed in vacuo. Column chromatography (9:1 chloroform:MeOH) gave the desired product as a colourless oil (0.34 g, 87 %). HRMS (ESI) = 303.0959 (M + Na)⁺. Calcd. for C₁₃H₁₆N₂O₅Na 303.0957. ¹H NMR (600 MHz, CDCl₃) δ 8.92 (br, 1 H), 7.47 (s, 1 H), 6.09 (t, *J* = 7.2 Hz, 1 H), 5.59 (m, 1 H), 4.70 (d, J = 1.8 Hz, 2 H), 4.61-4.57 (m, 1 H), 4.06 - 3.91 (m, 2 H), 2.71 (br, 1 H), 2.41-2.33 (m, 2 H), 2.16 (t, J = 1.8 Hz, 1 H), 1.89 (s, 3 H).

### N³-(2-Propynyl)-4'-thio-β-thymidine (6b).

The thionucleoside 6b was prepared according to the procedure for **6a** except using **3b** to give a colourless oil (51 mg, 86 %). HRMS (ESI) = 297.0911 (M + H)⁺. Calcd. for C₁₃H₁₇N₂O₄FS 297.0909. ¹H NMR (600 MHz, CDCl₃) δ 7.71 (d, *J*= 1.2 Hz, 1 H), 6.44 (t, *J* = 7.2 Hz, 1 H), 4.73 (d, *J* = 1.2 Hz, 2 H), 4.60-4.57 (m, 1 H), 4.01 (dd, *J* = 4.2 Hz, *J* = 11.4 Hz, 1 H), 3.85 (dd, *J* = 6.6 Hz, *J* = 11.4 Hz, 1 H), 3.53-3.51 (m, 1 H), 2.97 (br, 1 H), 2.51-2.47 (m, 1 H), 2.35-2.31 (m, 1 H), 2.17 (t, *J* = 1.2 Hz, 1 H), 1.98 (d, *J* = 1.2 Hz, 3 H).

### References

1. Kenny, L.; Coombes, R. C.; Vigushin, D. M.; Al-Nahhas, A.; Shousha, S.; Aboagye, E. O., Imaging early changes in proliferation at 1 week post chemotherapy: a pilot study in breast cancer patients with 3'-deoxy-3'-[(18)F]fluorothymidine positron emission tomography. *European Journal of Nuclear Medicine and Molecular Imaging* **2007**, In Press (Available online).
2. Leyton, J.; Alao, J. P.; Da Costa, M.; Stavropoulou, A. V.; Latigo, J. R.; Perumal, M.; Pillai, R.; He, Q.; Atadja, P.; Lam, E. W. F.; Workman, P.; Vigushin, D. M.; Aboagye, E. O., In vivo biological activity of the histone deacetylase inhibitor LAQ824 is detectable with 3'-deoxy-3'-[18F]fluorothymidine positron emission tomography. Cancer Research 2006, 66, (15), 7621-7629.
3. Leyton, J.; Latigo, J. R.; Perumal, M.; Dhaliwal, H.; He, Q.; Aboagye, E. O., Early detection of tumor response to chemotherapy by 3'-deoxy-3'-[18F]fluorothymidine positron emission tomography: the effect of cisplatin on a fibrosarcoma tumor model in vivo. Cancer Research 2005, 65, (10), 4202-4210.
4. Glaser, M.; Arstad, E., "Click Labeling" with 2-[18F]Fluoroethylazide for Positron Emission Tomography. Bioconjugate Chemistry 2007, 18, 989-993.
5. Inoue, N.; Kaga, D.; Minakawa, N.; Matsuda, A., Practical Synthesis of 2'-Deoxy-4'-thioribonucleosides: Substrates for the Synthesis of 4'-ThioDNA. Journal of Organic Chemistry 2005, 70, 8597-8600.

### Example 2

### Background

Here we describe the radiosynthesis of [¹⁸F]*N*³-((1-(2-Fluoroethyl)-1*H*-[1,2,3]-triazol-4-yl)methyl)-4'-thio-β-thymidine "[¹⁸F]FTT" and [¹⁸F]*N*³-((1-(2-Fluoroethyl)-1H-[1,2,3]-triazol-4-yl)methyl)thymidine "[¹⁸F]FOT"; the latter as a control radiotracer that may be less stable *in vivo.*

### Chemistry Discussion

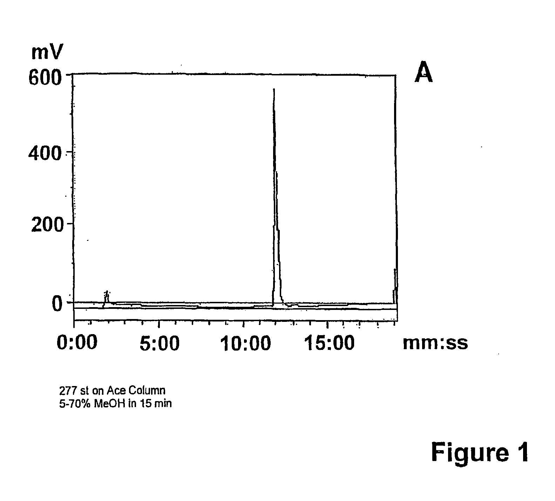

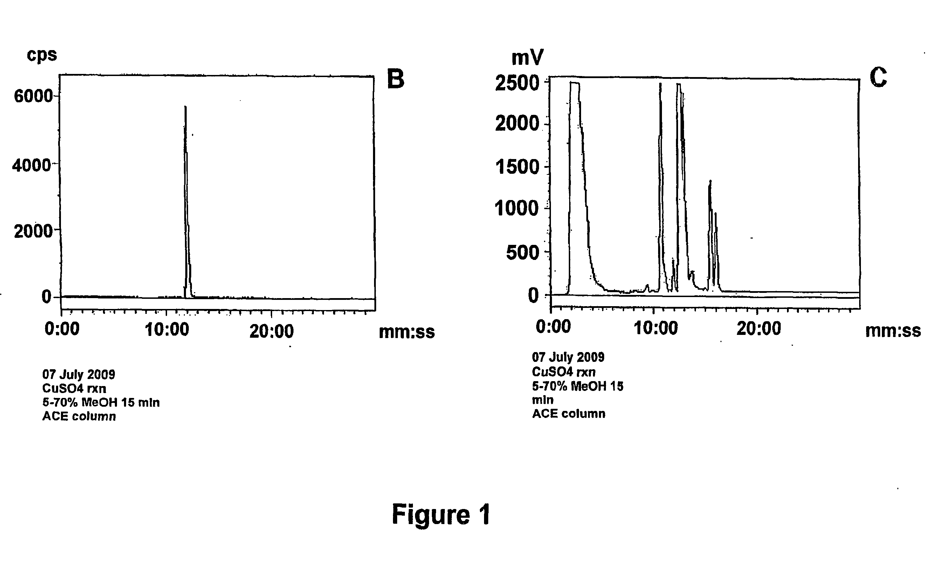

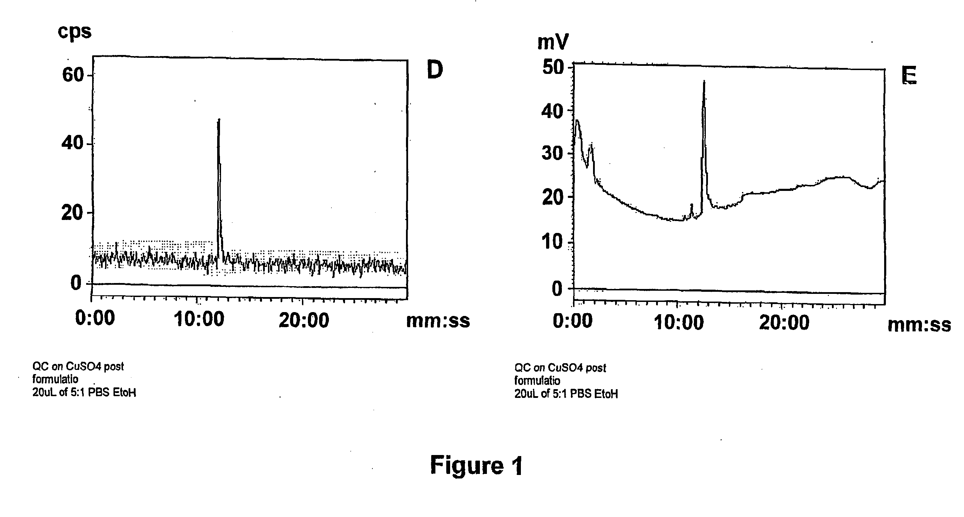

The radiosynthesis of [¹⁸F]FTT or [¹⁸F]FOT via a two step click chemistry procedure has been effected **(Scheme 6;** **Figure 1**). The procedure has been optimised in the following ways. Firstly, with respect to catalyst, copper sulfate/sodium ascorbate was found to be preferable to copper powder. The reaction was also studied at both ambient temperature and at 85°C, with the reaction at ambient temperature failing to reach completion (complete consumption of azide) in 30 min. In contrast, at the higher temperature (85°C) the reaction reached completion in 15 min., with no observable precursor degradation and as a consequence no further temperature optimisation was conducted.

Initially the procedure was effected with 2 mg of thio or oxo precursor; however, this was subsequently reduced to 1 mg with no observable impact on EOS radiochemical yield (5-10 % for **Scheme 6, 5a,** 9±4% for **Scheme 6, 5b).**

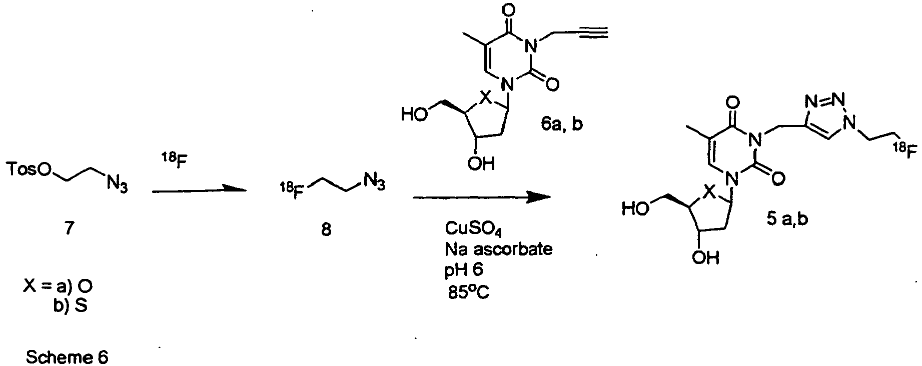

A representative specific activity measurement for the thio compound **5b** gave a value of 0.31 GBq/µmol; about 100-300-fold below the realistically achievable value. This anomaly is due to the presence of alkyne precursor in the formulated product. This value will be improved by improved HPLC peak separation between the product and precursor, leading to less carry over of precursor; using a higher level of starting radioactivity or the use of an azide scavenger, such as 2-(azidomethyl)naphthalene to remove unreacted alkyne precursor. This poor specific activity value will likely have a negative impact on the biological evaluation carried out to date.

### Biology Discussion

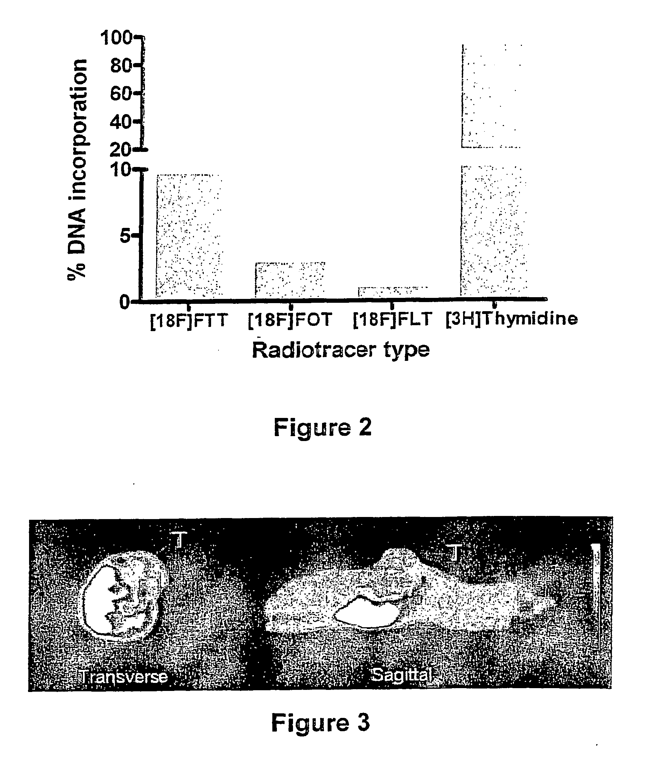

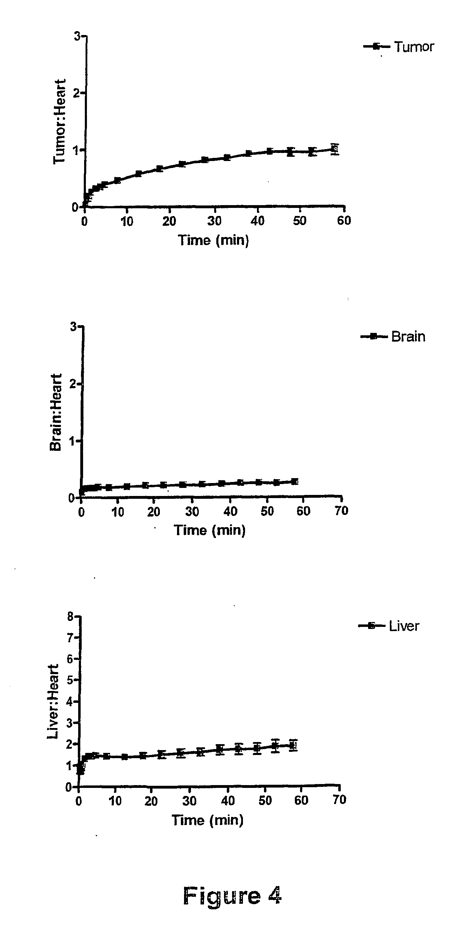

[¹⁸F]FTT was readily incorporated into tumour cells (HCT116 human colon cancer) in culture. Despite the current low specific radioactivity, we demonstrated (**Figure 2**) higher incorporation of [¹⁸F]FTT into the acid insoluble fraction (mainly DNA) of tumour cells compared [¹⁸F]FOT and [¹⁸F]FLT (current clinical radiotracer). All the fluorinated radiotracers had lower uptake into the acid insoluble fraction than the nature-identical radiotracer, [³H]thymidine. PET images demonstrated localisation in tumour, kidney, liver and the early part of the small intestines (**Figure 3**). [¹⁸F]FTT showed simple tissue pharmacokinetics (**Figure 4**).

### Experimental

### Chemistry:

Semi-preparative radio-HPLC purification was carried out on a Beckman System Gold (High Wycombe, UK) equipped with a Bioscan Flowcount FC-3400 PIN diode detector (Lablogic) and a linear UV-106 detector (wavelength 254 nm). Analyte separation was performed on an ACE C₁₈ 100 mm × 10 mm HPLC column using a mobile phase comprising of water and methanol (5 → 70 % methanol in 15 min.) delivered at a flow rate of 3 mL/min.

Analytical radio-HPLC and specific activity were measured on an Agilent 1100 series HPLC system (Agilent Technologies, Stockport, UK) equipped with a γ-RAM Model 3 gamma-detector (IN/US Systems Inc., Florida, USA) and the Laura 3 software (LabLogic, Sheffield, UK). Analyte separation was carried out on a Phenomenex Luna 5 µm C₁₈ 50 mm × 4.6 mm HPLC column using a mobile phase comprising of 10 mM K₂HPO₄ as solvent A and 7:3 acetonitrile/10 mM K₂HPO₄ (5 → 70 % methanol in 15 min.) delivered at a flow rate of 1 mL/min and wavelength 268 nm.

*General procedure for synthesis of [⁸F]FOT **5a** and [¹⁸F]FTT **5b**:* 2-[¹⁸F]Fluoroethyltosylate was synthesized according to an established procedure (15).

Under an atmosphere of nitrogen, a buffered solution (sodium phosphate buffer, pH 6.0, 250 mM) of sodium ascorbate (50 µl, 8.7 mg, 43.2 µmol) was added to a Wheaton vial (1 ml) containing an aqueous solution of copper(II) sulfate (50 µl, 1.7 mg pentahydrate, 7.0 µmol). After one min, a solution of N³-(2-Propynyl)thymidine **(Scheme 6, 6a)** or N³-(2-Propynyl)-4'-thio-β-thymidine **(Scheme 6, 6b)** (1.0 mg, -3 µmol) in DMF (25 µl) was added followed by distilled [¹⁸F]-2-fluoroethylazide (185-740 MBq) in acetonitrile (100 µl). The mixture was then heated at 85°C for 15 min., following which analytical radio-HPLC indicated complete consumption of azide. The mixture was allowed to cool to ambient temperature and water (400 µl) added, the resulting mixture was purified by preparative radio-HPLC. The isolated HPLC fraction was diluted with water (10 mL) and loaded onto a SepPak C18-light cartridge (Waters) that had been conditioned with ethanol (5 mL) and water (10 mL). The cartridge was subsequently flushed with water (5 mL) and [¹⁸F]FTT **Scheme 6, 5b** or [¹⁸F]FOT **Scheme 6, 5a** eluted with ethanol (0.1 mL fractions). The product fraction was diluted with PBS to provide an ethanol content of 10-15% (v/v).

### Acid insoluble fraction measurements:

Acid extraction was performed to separate radioactivity associated with small molecules, RNA, and proteins from that of DNA. HCT116 cells were cultured in triplicate in 100 mm dishes and the cells were used for DNA extraction at 24 hr. Briefly 5 ml of the assay medium containing 50 µCi of tracer was added to each dish and the dishes incubated at 37°C for 60 min. After incubation, the medium was removed, and cells were washed twice with ice-cold phosphate-buffered saline (PBS) and 100 µl aliquots of the washings were placed in counting tubes for counting. After washing, the cells were harvested by scraping, collected into counting tubes and then centrifuged at 1200 g at 4°C for 10 minutes. The cells were homogenized in 1mL of ice-cold 10% perchloric acid (HClO₄) and centrifuged. The acid-soluble fraction (supernatant) was removed and 100 µl aliquots were counted in a gamma counter. The radioactivity in the acid-insoluble precipitate (largely DNA) was counted separately.

### PET imaging studies:

All animal experiments were done by licensed investigators in accordance with the United Kingdom Home Office Guidance on the Operation of the Animal (Scientific Procedures) Act 1986 and within guidelines set out by the United Kingdom Coordinating Committee for Cancer Research's Ad hoc Committee on Welfare of Animals in Experimental Neoplasia. Both tumour-bearing (Balb/c nude mice) and non-tumour bearing (Balb/c mice) animals were used. Human colon, HCT116, tumours were grown in Balb/c *nu*/*nu* mice (Harlan) as previously reported. Tumour dimensions were measured continuously using a caliper and tumour volumes were calculated by the equation: volume = (π/6) × a × b × c, where a, b, and c represent three orthogonal axes of the tumour. Mice were used when their tumours reached approximately 100 mm³

Dynamic [¹⁸F]FTT imaging scans were carried out on a dedicated small animal PET scanner, Siemens Inveon PET module, Siemens Molecular Imaging Inc., Knoxville, USA). The features of this instrument have been described previously. For scanning the tail veins of vehicle- or drug-treated mice were cannulated after induction of anaesthesia (isofluorane/O₂/N₂O). The animals were placed within a thermostatically controlled jig (calibrated to provide a rectal temperature of ~37°C) and positioned prone in the scanner. [¹⁸F]FTT (2.96-3.7 MBq) was injected via the tail vein cannula and scanning commenced. Dynamic scans were acquired in list-mode format over a 60 min period as previously reported. The acquired data were then sorted into 0.5 mm sinogram bins and 19 time frames for image reconstruction, which was done by filtered back projection. Cumulative images of the dynamic data (0 to 30 min) were iteratively reconstructed (OSEM3D) and used for visualization of radiotracer uptake and to define the regions of interest (ROIs) with the Siemens Inveon Research Workplace software (three-dimensional ROIs were defined for each tumour). The count densities were averaged for all ROIs at each of the 19 time points to obtain a time versus radioactivity curve (TAC). Tumour TACs were normalized to that of whole body at each of the time points to obtain the normalized uptake value (NUV).

### Example 3

### [¹⁸F]FTT Radio-HPLC Metabolite Analysis

This example demonstrates that [¹⁸F]FTT is metabolically stable in mice, and therefore likely to be stable in humans.

### Experimental

[¹⁸F]FTT was produced using the method described above. HCT116 tumour bearing mice were injected intravenously via the lateral tail vein with [¹⁸F]FTT (3.7 MBq) and after 30 min post injection were sacrificied by exsanguination via cardiac puncture under isofluorane anesthesia and then tissue removed. Samples were then snap frozen using dry ice and stored until use. To plasma (0.2 ml) was added ice-cold acetonitrile (1.5 ml) and the sample centrifuged (3 min, 4°C, 15493 × g). The supernatant was then decanted and evaporated to dryness using a rotary evaporator (bath temperature 40°C). The sample was then redissolved in 10% ethanol in PBS (1.1 ml) and filtered through a 0.22 µm filter and then injected. Tumour, liver and kidney samples were homogenised in ice-cold acetonitile (1.5 ml) using an IKA Turrax homogeniser and then processed as for plasma samples. Urine samples were diluted with 10% ethanol in PBS (1.1 ml) and filtered through a 0.22 µm filter and then injected.

HPLC conditions: Agilent 1100 series HPLC system (Agilent Technologies, Stockport, UK) equipped with a γ-RAM Model 3 gamma-detector (IN/US Systems Inc., Florida, USA) and the Laura 3 software (LabLogic, Sheffield, UK). Analyte separation was carried out on a Waters Bondapak 10 µm C₁₈ 300 mm × 7.8 mm HPLC column using a mobile phase comprising of 10 mM K₂HPO₄ as solvent A and 7:3 acetonitrile/10 mM K₂HPO₄ as solvent B (30 % solvent B, isocratic) delivered at a flow rate of 3 ml/min and wavelength 268 nm.

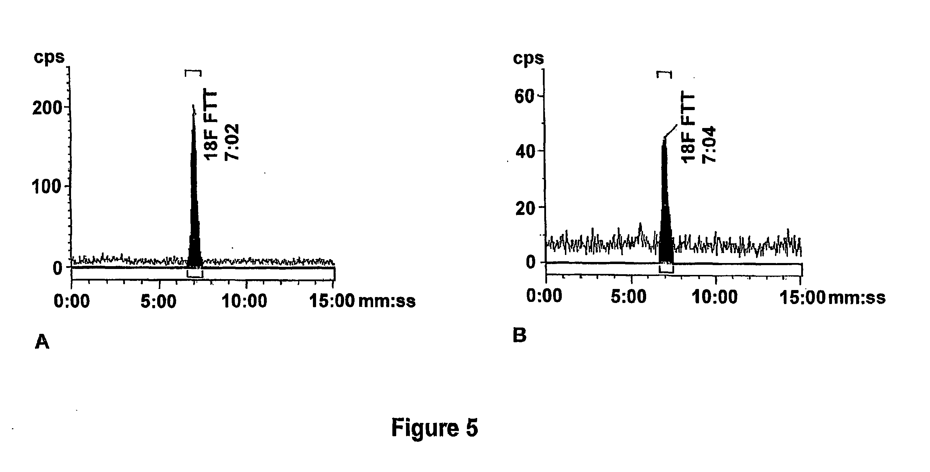

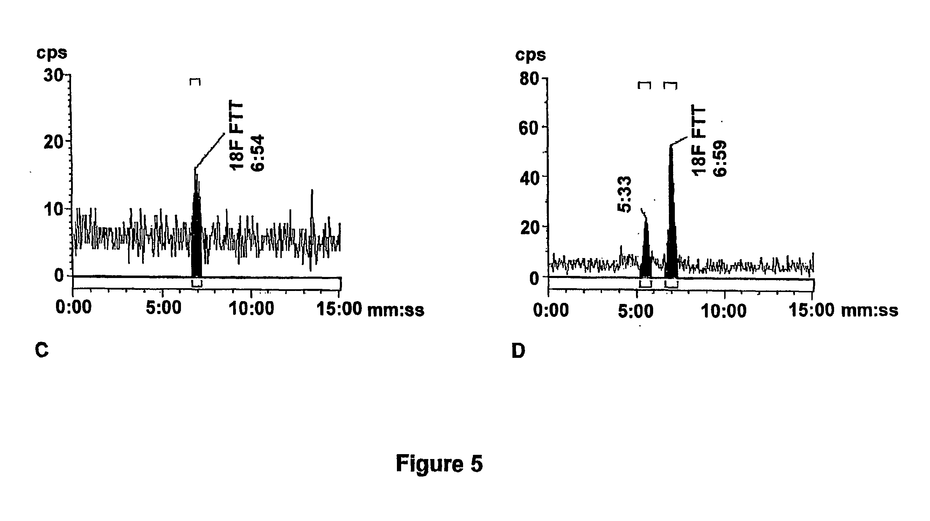

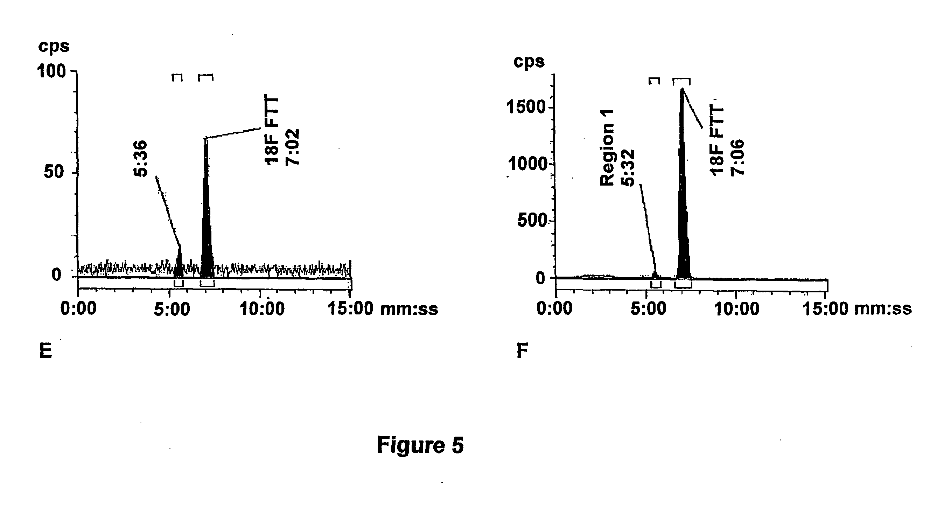

The results of the [¹⁸F]FTT radio-HPLC metabolite analysis are displayed in Figure 5.

## Claims

1. A 4'-thio nucleoside that is a derivative of 4'-thiothymidine or 4'-thio-2'-deoxyuridine comprising a positron or single photon emitting radioisotope or corresponding non-radioactive isotope attached via a triazole link to the N-3 position, optionally wherein the 4'-thio nucleoside is in the form of a salt.

2. The 4'-thio nucleoside of Claim 1 wherein the positron emitting radioisotope is [¹⁸F] or
the corresponding non-radioactive isotope is F.

3. The 4'-thio nucleoside of Claim 1 wherein the positron or single photon emitting radioisotope is ¹¹C, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I,
¹³¹I, or ²⁰¹Tl; or wherein the radioisotope is ³H, ¹⁴C or ³⁵S.

4. The 4'-thio nucleoside of Claims 1, 2 or 3 having the structure: 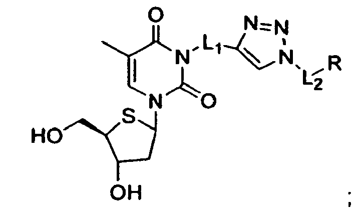 or 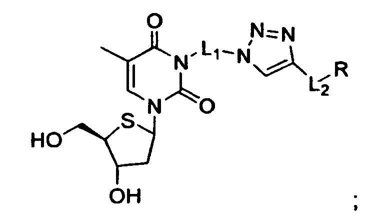 wherein R is a reporter moiety with a PET/SPECT radioisotope comprising ¹¹C, ¹⁸F, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I, ²⁰¹Tl, ³H, ¹⁴C or ³⁵S; and
wherein L₁ and L₂ are each independently a C₁₋₃₀ hydrocarbon chain which may be branched or straight chain, optionally wherein L₁ and/or L₂ comprise a hydrocarbon chain substituted with 1 to 15 heteroatoms, optionally wherein the heteroatom is oxygen, nitrogen or sulfur;
or having the structure: 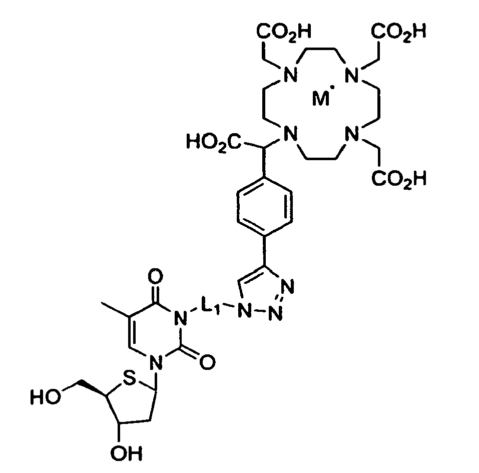 wherein M^{*} is ⁶¹Cu ⁶⁴Cu, ⁶⁷Cu, ⁶⁷Ga, ⁶⁸Ga, ¹¹¹In, ²⁰¹Tl, ^{94m}Tc, ^{99m}Tc, ³H, ¹⁴C or ¹²⁵I;
and
wherein L₁ is a C₁₋₃₀ hydrocarbon chain which may be branched or straight chain, optionally wherein L₁ comprises a hydrocarbon chain substituted with 1 to 15 heteroatoms, optionally wherein the heteroatom is oxygen, nitrogen or sulfur.

5. The 4'-thio nucleoside of Claim 4 wherein the hydrocarbon chain further comprises alkenyl, alkynyl, cycloalkyl, aryl, polyaryl or heteroaryl units, optionally wherein the cycloalkyl ring comprises 3 to 12 carbon atoms, optionally wherein the cycloalkly ring is substituted with 1 to 5 heteroatoms, optionally wherein the heteroatom is oxygen, nitrogen or sulphur; or optionally wherein the heteroaryl unit is fused to a further aryl or heteroaryl unit.

6. The 4'-thio nucleoside of Claim 5 wherein the aryl, polyaryl or heteroaryl unit are substituted with one or more C₁₋₅ alkyl, C₃₋₆ alkenyl, C₃₋₆ alkynyl, halogen (fluorine, chlorine, bromine, iodine), -CF₃, nitro, amino, hydroxyl, aldehyde, COOC₁₋₅ alkyl, -OC₁₋₅ alkyl, CONHC₁₋₅ alkyl, -NHCOC₁₋₅ alkyl and -NHSO₂C₁₋₅ alkyl.

7. The 4'-thio nucleoside of Claims 1, 2 or 4 having a structure selected from the following structures: 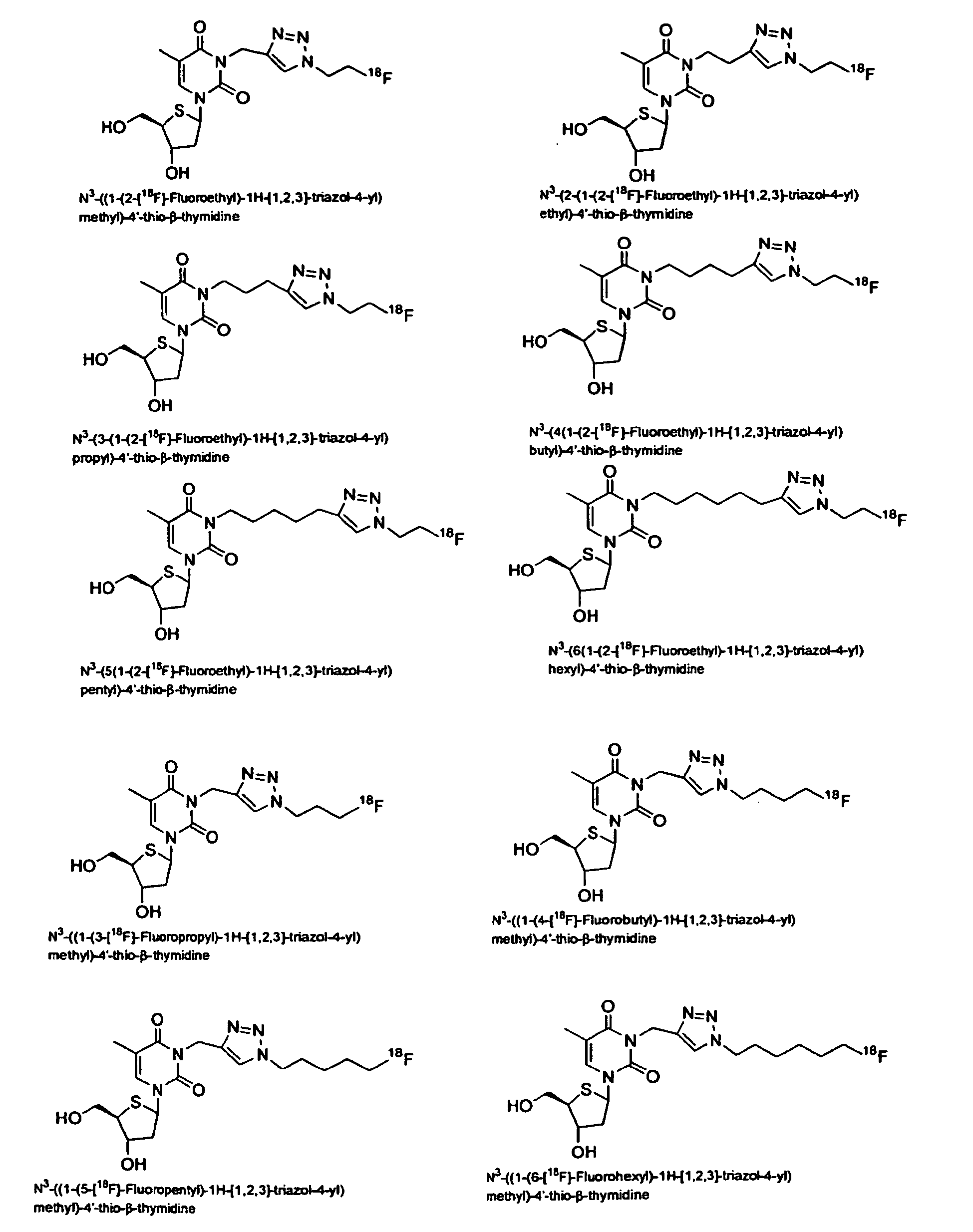 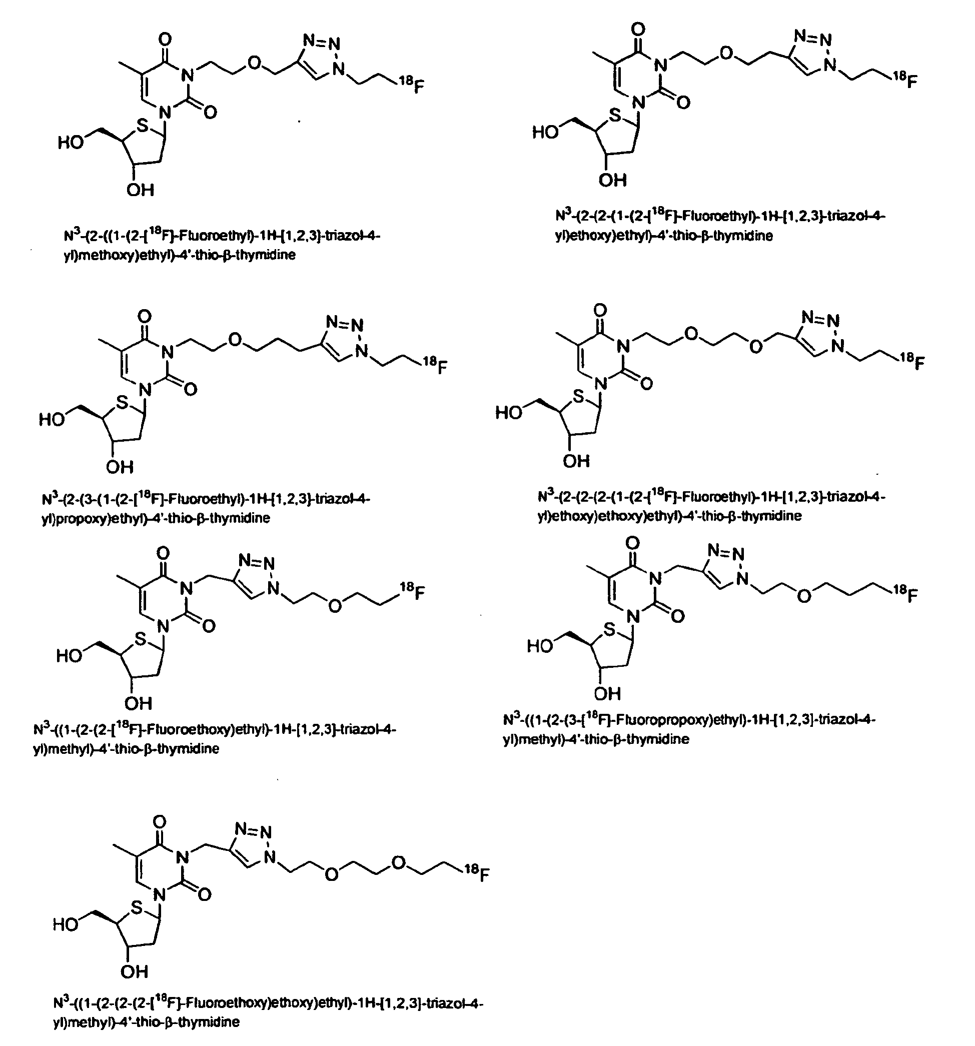 or having a structure selected from the following structures: 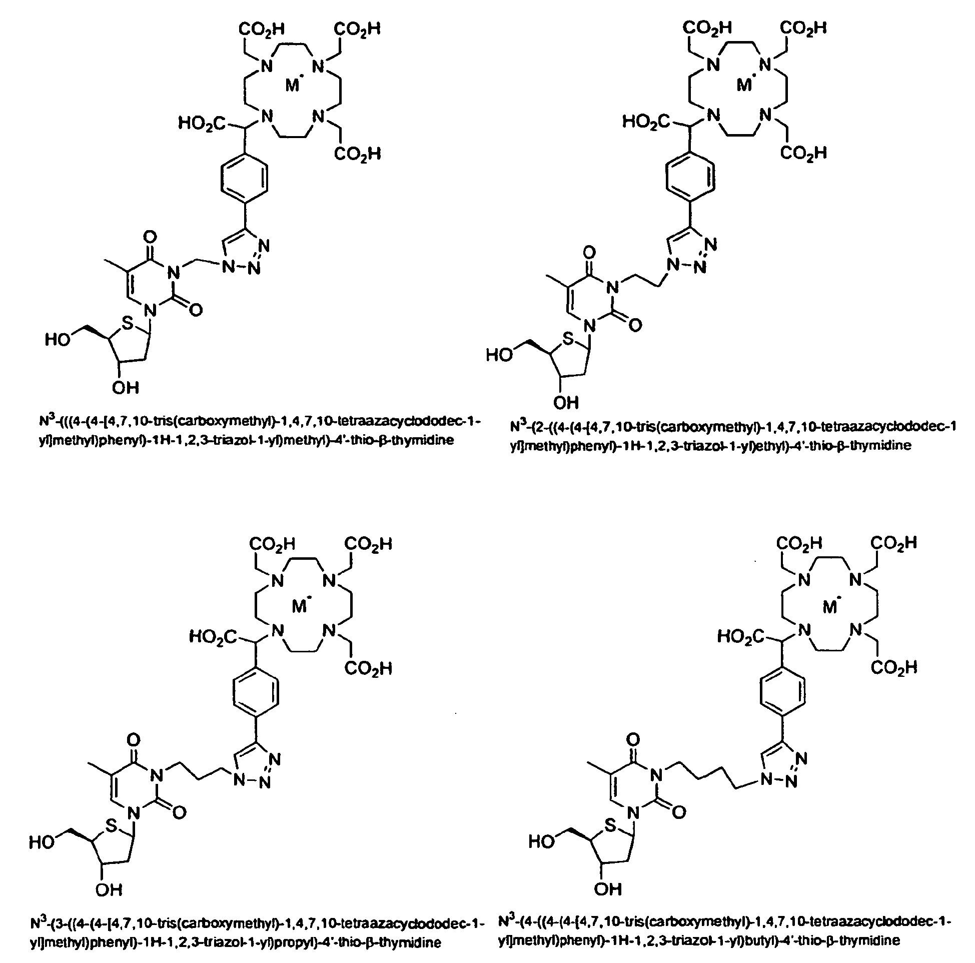 wherein M^{*} is ⁶¹Cu, ⁶⁴Cu, ⁶⁷Cu, ⁶⁷Ga, ⁶⁸Ga, ^{94m}Tc, ^{99m}Tc, ¹¹¹In, ²⁰¹Tl, ³H, ¹⁴_{C} or ¹²⁵I.

8. A derivative of 4'-thiothymidine or 4'-thio-2'-deoxyuridine comprising a terminal alkyne attached via the N-3 position; or a derivative of 4'-thiothymidine or 4'-thio-2'-deoxyuridine comprising a terminal azide attached via the N-3 position.

9. A method for preparing a 4'-thio nucleoside according to any one of the preceding claims comprising the step of exposing a derivative of 4'-thiothymidine or 4'-thio-2'-deoxyuridine comprising a terminal alkyne attached via the N-3 position to a radiolabelled (or corresponding non-radiolabelled) compound comprising an azide group; or the step of exposing a derivative of 4'-thiothymidine or 4'-thio-2'-deoxyuridine comprising an azide group attached via the N-3 position to a radiolabelled (or corresponding non-radiolabelled) compound comprising a terminal alkyne group.

10. A pharmaceutical composition comprising a 4'-thio nucleoside according to any one of Claims 1 to 7 and a pharmaceutically acceptable carrier.

11. A 4'-thio nucleoside according to any one of Claims 1 to 7 for use in medicine.

12. A 4'-thio nucleoside according to any one of Claims 1 to 7 for use in aiding imaging, prognosis, diagnosis or analysis of response to treatment of a proliferative disease, optionally wherein the proliferative disease is cancer, rheumatoid arthritis, endometrial hyperplasia, vascular restinosis or sclerosis.

13. Use of a 4'-thio nucleoside according to any one of Claims 1 to 7 in the manufacture of a medicament for use in imaging, prognosis, diagnosis, selection for treatment or analysis of response to treatment of a proliferative disease, optionally wherein the proliferative disease is cancer, rheumatoid arthritis, endometrial hyperplasia, vascular restinosis or sclerosis.

14. A kit of parts comprising a derivative of 4'-thiothymidine or 4'-thio-2'-deoxyuridine comprising a terminal alkyne or azide attached via the N-3 position and a radiolabelled (or corresponding non-radiolabelled) compound comprising an azide group or terminal alkyne group.

15. The derivative of Claim 8 or kit of parts of Claim 14 wherein the derivative is attached to a solid support through one or both of the hydroxyl groups on the sugar ring, wherein the solid support is a solid phase resin which is functionalised with an alkyl, trityl or acyl group, optionally wherein the resin is polystyrene, polyamide, polyacrylamide, or glass or silicon coated with a polymer, optionally wherein the solid phase resin is in the form of small discrete particles such as beads or coated to the inner surface of a cartridge or on the lining of a reaction vessel.

16. Use of a 4'-thio nucleoside according to any one of Claims 1 to 7 for *in vitro* analysis of proliferation and anti-proliferative activity.

## Patentansprüche

1. 4'-Thionucleosid, das ein Derivat von 4'-Thiothymidin oder 4'-Thio-2'-desoxyuridin ist, umfassend ein Positronen oder Einzelphotonen emittierendes Radioisotop oder ein entsprechendes nichtradioaktives Isotop, das über eine Triazol-Verknüpfung an der N-3-Position angebunden ist, wobei das 4'-Thionucleosid optional in der Form eines Salzes vorliegt.

2. 4'-Thionucleosid nach Anspruch 1, wobei das Positronen emittierende Radioisotop [¹⁸F] ist oder das entsprechende nichtradioaktive Isotop F ist.

3. 4'-Thionucleosid nach Anspruch 1, wobei das Positronen oder Einzelphotonen emittierende Radioisotop ¹¹C, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I oder ²⁰¹Tl ist oder wobei das Radioisotop ³H, ¹⁴C oder ³⁵S ist.

4. 4'-Thionucleosid nach den Ansprüchen 1, 2 oder 3 mit der Struktur: 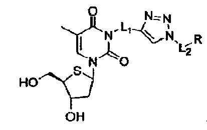 oder 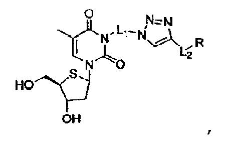 wobei R eine Reportereinheit mit einem PET/SPECT-Radioisotop, das ¹¹C, ¹⁸F, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I, ²⁰¹Tl, ³H, ¹⁴C oder ³⁵S umfasst, ist; und
wobei L₁ und L₂ jeweils unabhängig voneinander für eine C₁₋₃₀-Kohlenwasserstoffkette, die verzweigt oder geradkettig sein kann, stehen, wobei L₁ und/oder L₂ optional eine mit 1 bis 15 Heteroatomen substituierte Kohlenwasserstoffkette umfassen, wobei das Heteroatom optional Sauerstoff, Stickstoff oder Schwefel ist;
oder mit der Struktur: 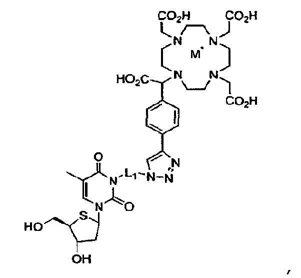 wobei M^{*} für ⁶¹Cu, ⁶⁴Cu, ⁶⁷Cu, ⁶⁷Ga, ⁶⁸Ga, ¹¹¹In, ²⁰¹Tl, ^{94m}Tc, ^{99m}Tc, ³H, ¹⁴C oder ¹²⁵I steht; und
wobei L₁ für eine C₁₋₃₀-Kohlenwasserstoffkette, die verzweigt oder geradkettig sein kann, steht, wobei L₁ optional eine mit 1 bis 15 Heteroatomen substituierte Kohlenwasserstoffkette umfasst, wobei das Heteroatom optional Sauerstoff, Stickstoff oder Schwefel ist.

5. 4'-Thionucleosid nach Anspruch 4, wobei die Kohlenwasserstoffkette ferner Alkenyl-, Alkinyl-, Cycloalkyl-, Aryl-, Polyaryl- oder Heteroaryleinheiten umfasst, wobei der Cycloalkylring optional 3 bis 12 Kohlenstoffatome umfasst, wobei der Cycloalkylring optional mit 1 bis 5 Heteroatomen substituiert ist, wobei das Heteroatom optional Sauerstoff, Stickstoff oder Schwefel ist oder wobei die Heteroaryleinheit optional an eine weitere Aryl- oder Heteroaryleinheit ankondensiert ist.

6. 4'-Thionucleosid nach Anspruch 5, wobei die Aryl-, Polyaryl- oder Heteroaryleinheiten mit einer oder mehreren Gruppen von C₁₋₅-Alkyl, C₃₋₆-Alkenyl, C₃₋₆-Alkinyl, einem Halogen (Fluor, Chlor, Brom, Iod), -CF₃, Nitro, Amino, Hydroxyl, Aldehyd, COO-C₁₋₅-Alkyl, -O-C₁₋₅-Alkyl, CONH-C₁₋₅-Alkyl, -NHCO-C₁₋₅-Alkyl und -NHSO₂-C₁₋₅-Alkyl substituiert sind.

7. 4'-Thionucleosid nach den Ansprüchen 1, 2 oder 4 mit einer Struktur, die aus den folgenden Strukturen ausgewählt ist: 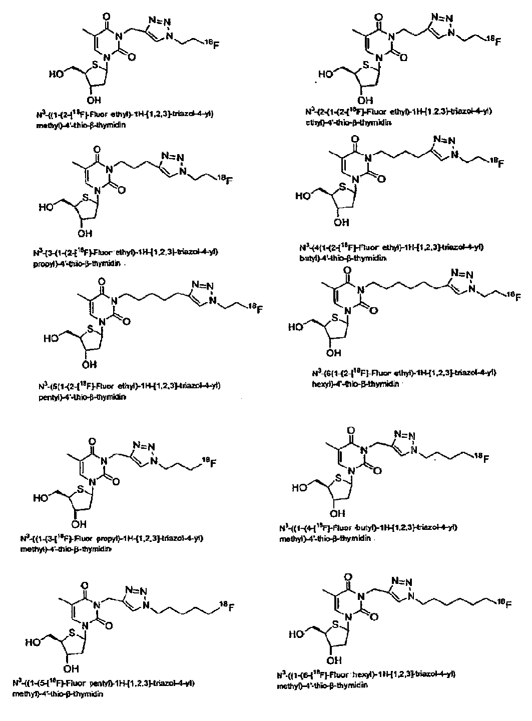 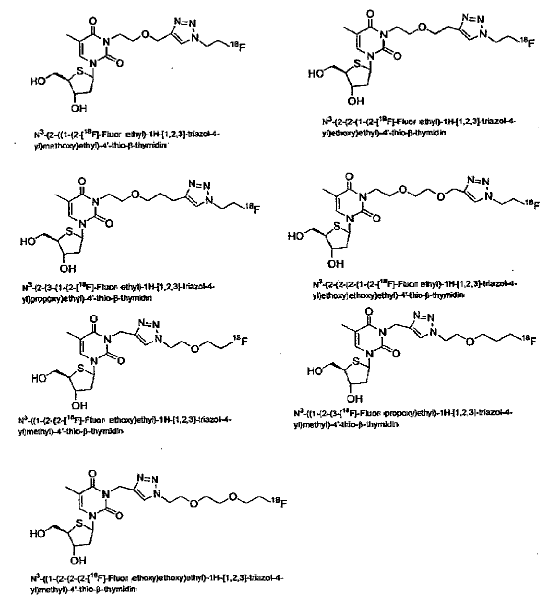 oder mit einer Struktur, die aus den folgenden Strukturen ausgewählt ist: 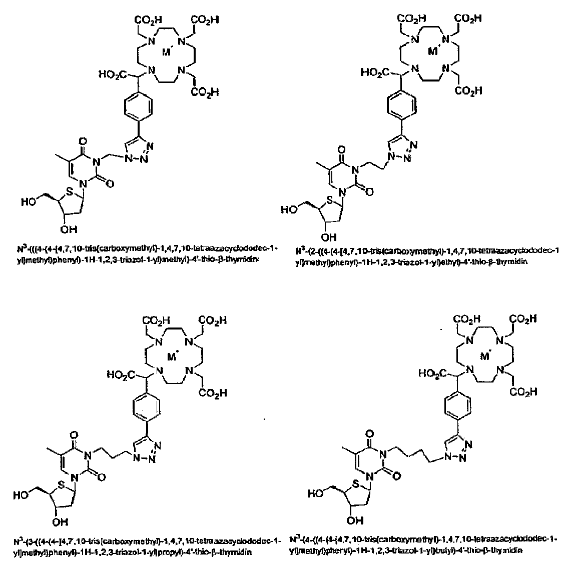 wobei M^{*} für ⁶¹Cu, ⁶⁴Cu, ⁶⁷Cu, ⁶⁷Ga, ⁶⁸Ga, ^{94m}Tc, ^{99m}Tc, ¹¹¹In, ²⁰¹Tl, ³H, ¹⁴C oder ¹²⁵I steht.

8. Derivat von 4'-Tniothymidin oder 4'-Thio-2'-desoxyuridin, umfassend ein terminales Alkin, das über die N-3-Position angebunden ist; oder ein Derivat von 4'-Thiothymidin oder 4'-Thio-2'-desoxyuridin, umfassend ein terminales Azid, das über die N-3-Position angebunden ist.

9. Verfahren zur Herstellung eines 4'-Thionucleosids nach einem der vorhergehenden Ansprüche, umfassend die Stufe des Einwirkons einer radioaktiv markierten (oder entsprechenden nicht-radioaktiv-markierten) Verbindung, die eine Azidgruppe umfasst, auf ein Derivat von 4'-Thiothymidin oder 4'-Thio-2'-desoxyuridin, das ein über die N-3-Position angebundenes terminales Alkin umfasst, oder die Stufe des Einwirkens einer radioaktiv markierten (oder entsprechenden nicht-radioaktiv-markierten) Verbindung, die eine terminale Alkingruppe umfasst, auf ein Derivat von 4'-Thiothymidin oder 4'-Thio-2'-desoxyuridin, das eine über die N-3-Position angebundene Azidgruppe umfasst.

10. Pharmazeutische Zusammensetzung, die ein 4'-Thionucleosid nach einem der Ansprüche 1 bis 7 und einen pharmazeutisch akzeptablen Träger umfasst.

11. 4'-Thionucleosid nach einem der Ansprüche 1 bis 7 zur Verwendung in der Medizin.

12. 4'-Thionucleosid nach einem der Ansprüche 1 bis 7 zur Verwendung zur Unterstützung der Bildgebung, Prognose, Diagnose oder Analyse des Ansprechens auf eine Behandlung einer proliferativen Erkrankung, wobei die proliferative Erkrankung optional eine Krebserkrankung, rheumatoide Arthritis, Endometriumhyperplasie, vaskuläre Stenose oder Sklerose ist.

13. Verwendung eines 4'-Thionucleosids nach einem der Ansprüche 1 bis 7 bei der Herstellung eines Medikaments zur Verwendung zur Bildgebung, Prognose, Diagnose, Auswahl für eine Behandlung oder Analyse des Ansprechens auf eine Behandlung einer proliferativen Erkrankung, wobei die proliferative Erkrankung optional eine Krebserkrankung, rheumatoide Arthritis, Endometriumhyperplasie, vaskuläre Stenose oder Sklerose ist.

14. Kit aus Teilen, das ein Derivat von 4'-Thiothymidin oder 4'-Thio-2'-desoxyuridin, das ein über die N-3-Position angebundenes terminales Alkin oder Azid umfasst, und eine radioaktiv markierte (oder entsprechende nicht-radioaktivmarkierte) Verbindung, die eine Azidgruppe oder eine terminale Alkingruppe umfasst, umfasst.

15. Derivat nach Anspruch 8 oder Kit aus Teilen nach Anspruch 14, wobei das Derivat über eine oder beide der Hydroxylgruppen an dem Zuckerring an einen festen Träger gebunden ist, wobei der feste Träger ein Festkörperharz ist, das mit einer Alkyl-, Trityl- oder Acylgruppe funktionalisiert ist, wobei das Harz optional ein Polystyrol, Polyamid, Polyacrylamid oder mit einem Polymer beschichtetes Glas oder Silicium ist, wobei das Festkörperharz optional in der Form kleiner diskreter Teilchen, beispielsweise von Perlen, vorliegt oder auf die innere Oberfläche einer Kartusche oder auf die Auskleidung eines Reaktionsgefäßes aufgetragen ist.

16. Verwendung eines 4'-Thionucleosids nach einem der Ansprüche 1 bis 7 zur In-vitro-Analyse von Proliferation und antiproliferativer Aktivität.

## Revendications

1. 4'-Thionucléoside qui est un dérivé d'une 4'-thiothymidine ou d'une 4'-thio-2'-désoxyuridine comprenant un radio-isotope émetteur de positon ou de photon unique ou un isotope non radioactif correspondant lié par l'intermédiaire d'un lien triazole à la position N-3, éventuellement dans lequel le 4'-thionucléoside se présente sous la forme d'un sel.

2. 4'-Thionucléoside selon la revendication 1, dans lequel le radio-isotope émetteur de positon est le [¹⁸F] ou l'isotope non radioactif correspondant est F.

3. 4'-Thionucléoside selon la revendication 1, dans lequel le radio-isotope émetteur de positon ou de photon unique est ¹¹C, ⁶¹Cu, ⁶⁴Cu, ⁶⁷Cu, ⁶⁷Ga, ⁶⁸Ga, ⁷⁵Br, ^{94m}Tc, ^{99m}TC, ¹¹¹In, ¹²³I, ¹²⁴I, ¹²⁵I, ¹³¹I ou ²⁰¹Tl ; ou dans lequel le radio-isotope est ³H, ¹⁴C ou ³⁵S.

4. 4'-Thionucléoside selon la revendication 1, 2 ou 3 répondant à la structure : 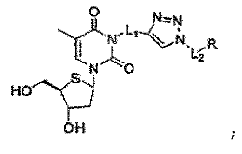 ou 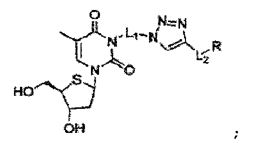 dans lequel R est un fragment rapporteur avec un radio-isotope TEP/TEPU comprenant ¹¹C, ¹⁹F, ⁶¹Cu, ⁶⁴Cu, ⁶⁷Cu, ⁶⁷Ga, ⁶⁸Ga, ⁷⁵Br, ⁷⁶Br, ^{94m}Tc, ^{99m}Tc, ¹¹¹In, ¹²³I, ¹²⁴I, ¹²⁵I, ¹³¹I, ²⁰¹Tl, ³H, ¹⁴C ou ³⁵S ; et
dans lequel L₁ et L₂ sont chacun indépendamment une chaîne hydrocarbure en C₁ à C₃₀ qui peut être une chaîne ramifiée ou droite, éventuellement dans lequel L₁ et/ou L₂ comprennent une chaîne hydrocarbure substituée par 1 à 15 hétéroatomes, éventuellement dans lequel l'hétéroatome est l'oxygène, l'azote ou le soufre ;
ou répondant à la structure : 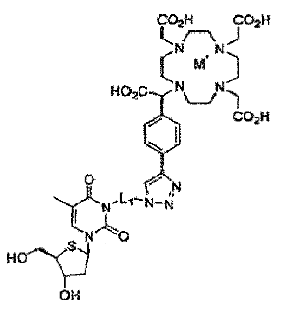 dans lequel M^{*} est ⁶¹Cu, ⁶⁴Cu, ⁶⁷Ga, ⁶⁸Ga, ¹¹¹In, ²⁰¹Tl, ^{94m}Tc, ^{99m}Tc, ³H, ¹⁴C ou ¹²⁵I ; et
dans lequel L₁ est une chaîne hydrocarbure en C₁ à C₃₀ qui peut être une chaîne ramifiée ou droite, éventuellement dans lequel L₁ comprend une chaîne hydrocarbure substituée par 1 à 15 hétéroatomes, éventuellement dans lequel l'hétéroatome est l'oxygène, l'azote ou le soufre.

5. 4'-Thionucléoside selon la revendication 4, dans lequel la chaîne hydrocarbure comprend en outre des motifs alcényle, alcynyle, cycloalkyle, aryle, polyaryle ou hétéroaryle, éventuellement dans lequel le cycle cycloalkyle comprend 3 à 12 atomes de carbone, éventuellement dans lequel le cycle cycloalkyle est substitué par 1 à 5 hétéroatomes, éventuellement dans lequel l'hétéroatome est l'oxygène, l'azote ou le soufre ; ou éventuellement dans lequel le motif hétéroaryle est fusionné à un motif aryle ou hétéroaryle supplémentaire.

6. 4'-Thionucléoside selon la revendication 5, dans lequel le motif aryle, polyaryle ou hétéroaryle est substitué par un ou plusieurs des éléments suivants : un alkyle en C₁ à C₅, un alcényle en C₃ à C₆, un alcynyle en C₃ à C₆, un halogène (fluor, chlore, brome, iode), -CF₃, un nitro, un amino, un hydroxyle, un aldéhyde, COO-alkyle en C₁ à C₅, -O-alkyle en C₁ à C₅, CONH-alkyle en C₁ à C₅, NHCO-alkyle en C₁ à C₅ et -NHSO₂-alkyle en C₁ à C₅.

7. 4'-Thionucléoside selon la revendication 1, 2 ou 4, dont la structure est choisie parmi les structures suivantes : 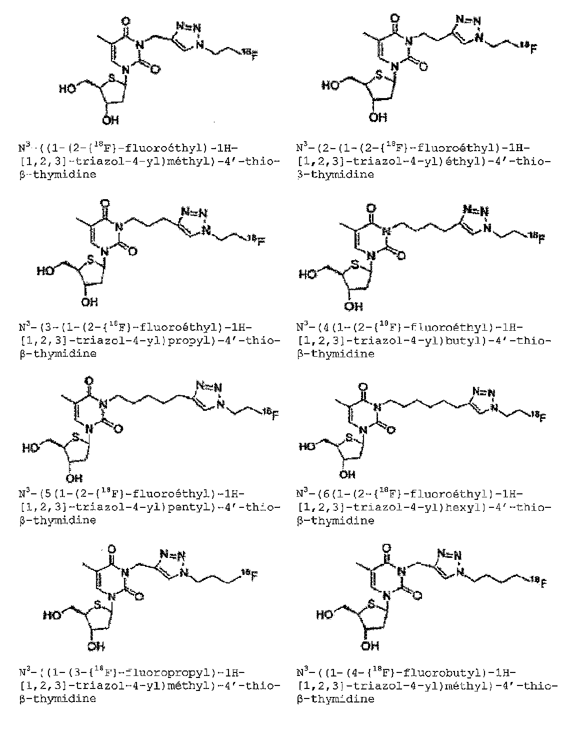 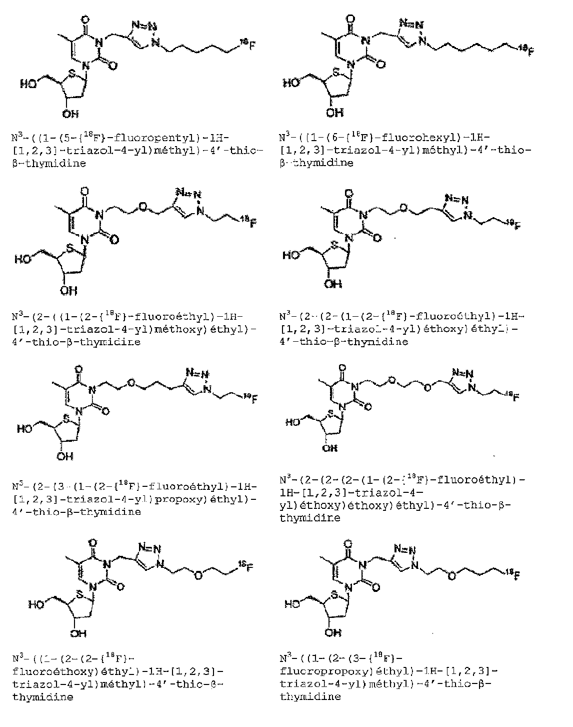 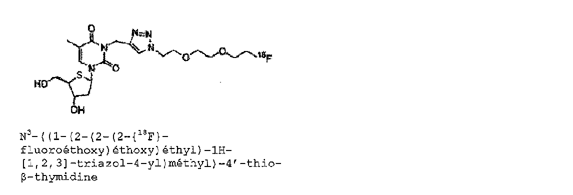 ou dont la structure est choisie parmi les structures suivantes : 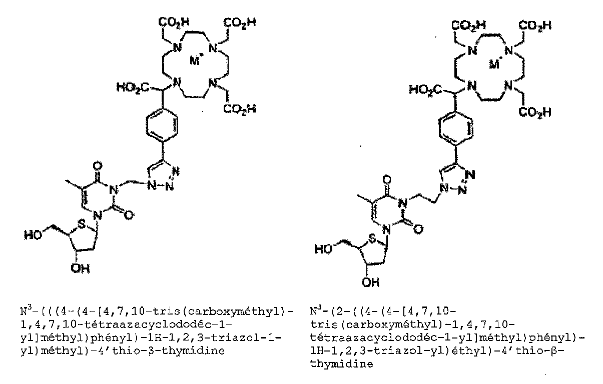 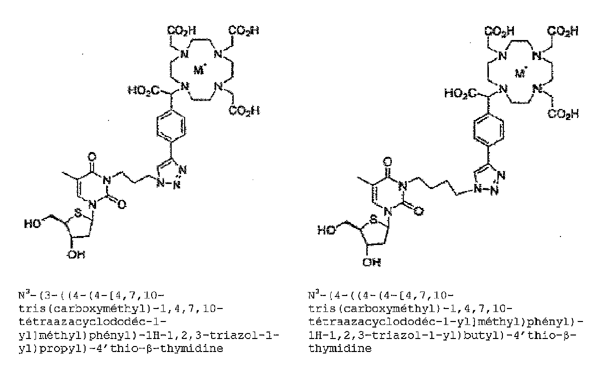 dans lequel M* est ⁶¹Cu, ⁶⁴Cu, ⁶⁷Cu, ⁶⁷Ga, ⁶⁸Ga, ^{99m}Tc, ^{99m}Tc, ¹¹¹In, ²⁰¹Tl, ³H, ¹⁴C ou ¹²⁵I.

8. Dérivé de 4'-thiothymidine ou de 4'-thio-2'-désoxyuridine comprenant un alcyne terminal lié via la position N-3 ; ou dérivé de 4'-thiothymidine ou de 4'-thio-2'-désoxyuridine comprenant un azoture terminal lié via la position N-3.

9. Procédé de préparation d'un 4'-thionucléoside selon l'une quelconque des revendications précédentes, comprenant l'étape d'exposition d'un dérivé de 4'-thiothymidine ou de 4'-thio-2'-désoxyuridine comprenant un alcyne terminal lié via la position N-3 à un composé radiomarqué (ou non radiomarqué correspondant) comprenant un groupe azoture ; ou l'étape d'exposition d'un dérivé de 4'-thiothymidine ou de 4'-thio-2'-désoxyuridine comprenant un groupe azoture lié via la position N-3 à un composé radiomarqué (ou non radiomarqué correspondant) comprenant un groupe alcyne terminal.

10. Composition pharmaceutique comprenant un 4'-thionucléoside selon l'une quelconque des revendications 1 à 7 et un véhicule pharmaceutiquement acceptable.

11. 4'-Thionucléoside selon l'une quelconque des revendications 1 à 7, à utiliser en médecine.

12. 4'-Thionucléoside selon l'une quelconque des revendications 1 à 7, à utiliser en aide à l'imagerie, le pronostic, le diagnostic ou l'analyse de la réponse au traitement d'une maladie proliférative, éventuellement dans lequel la maladie proliférative est le cancer, la polyarthrite rhumatoïde, l'hyperplasie endométriale, la resténose vasculaire ou la sclérose.

13. Utilisation d'un 4'-thionucléoside selon l'une quelconque des revendications 1 à 7, dans la fabrication d'un médicament à utiliser en aide à l'imagerie, le pronostic, le diagnostic, le choix du traitement ou l'analyse de la réponse au traitement d'une maladie proliférative, éventuellement dans laquelle la maladie proliférative est le cancer, la polyarthrite rhumatoïde, l'hyperplasie endométriale, la resténose vasculaire ou la sclérose.

14. Kit de pièces comprenant un dérivé de 4'-thiothymidine ou de 4'-thio-2'-désoxyuridine comprenant un alcyne terminal ou un azoture terminal lié via la position N-3 et un composé radiomarqué (ou non radiomarqué correspondant) comprenant un groupe azoture ou un groupe alcyne terminal.

15. Dérivé selon la revendication 8 ou kit de pièces selon la revendication 14, dans lequel le dérivé est lié à un support solide par l'intermédiaire de l'un ou des deux groupes hydroxyle sur le cycle sucre, dans lequel le support solide est une résine en phase solide qui est fonctionnalisée par un groupe alkyle, trityle ou acyle, éventuellement dans lequel la résine est un polystyrène, un polyamine, un polyacrylamide, ou du verre ou du silicium revêtu d'un polymère, éventuellement dans lequel la résine en phase solide se présente sous la forme de petites particules distinctes telles que des billes ou revêtues sur la surface interne d'une cartouche ou sur la chemise d'une cuve de réaction.

16. Utilisation d'un 4'-thionucléoside selon l'une quelconque des revendications 1 à 7, pour l'analyse in vitro de la prolifération et de l'activité anti-proliférative.
